# EUROPEAN PATENT SPECIFICATION

(11) **EP 4 510 919 B1**
(45) Date of publication and mention of the grant of the patent: **19.08.2026**
(21) Application number: 22725954.6
(22) Date of filing: 18.04.2022
(51) Int. Cl.: A61B 5/024, A61B 5/00, G06F 3/042, G06F 3/01

(54) **WEARABLE COMPUTING DEVICE HAVING OPTICAL SENSORS TO INDIRECTLY DETERMINE A LOCATION OF A FORCE APPLIED TO A USER INTERFACE**
TRAGBARE RECHNERVORRICHTUNG MIT OPTISCHEN SENSOREN ZUR INDIREKTEN BESTIMMUNG EINER POSITION EINER AUF EINE BENUTZERSCHNITTSTELLE AUSGEÜBTEN KRAFT
DISPOSITIF INFORMATIQUE PORTABLE AYANT DES CAPTEURS OPTIQUES POUR DÉTERMINER INDIRECTEMENT UNE LOCALISATION D'UNE FORCE APPLIQUÉE À UNE INTERFACE UTILISATEUR

(43) Date of publication of application: 26.02.2025
(73) Proprietor: Google LLC, Mountain View, CA 94043 (US)
(72) Inventor: SHIN, Dongeek, Mountain View, California 94043 (US); COLACO, Andrea B., Mountain View, CA 94043 (US)
(74) Representative: Maikowski & Ninnemann Patentanwälte Partnerschaft mbB
(86) International application number: PCT/US2022/025198
(87) International publication number: WO 2023/204795

(56) References cited:
- US-A1- 2014 275 852
- US-A1- 2017 251 935
- US-A1- 2020 113 442
- US-A1- 2020 245 900
- US-A1- 2021 330 261
- US-B1- 10 874 348

## Description

### FIELD

The disclosure relates generally to wearable computing devices. More particularly, the disclosure relates to wearable computing devices which can determine a location of a force applied to a user interface of the wearable computing device using optical sensors.

### BACKGROUND

Wearable computing devices (e.g., wrist watches) can include a display to display content (e.g., time, date, functions of the wearable computing device, etc.) to a user. Some wearable computing devices can gather data regarding activities performed by the user, or regarding the user's physiological state. Such data may include data representative of the ambient environment around the user or the user's interaction with the environment. For example, the data can include motion data regarding the user's movements and/or physiological data obtained by measuring various physiological characteristics of the user, such as heart rate, perspiration levels, and the like. Some wearable computing devices, such as disclosed in US 10,874,348 B1, US 204/0275852 A1, and US 2017/0251935 A1, may include a touch-sensitive display, while other wearable computing devices may include a non-touch-sensitive display.

### SUMMARY

The proposed solution relates to a wearable computing device of claim and a method of claim 11.

Aspects and advantages of embodiments of the disclosure will be set forth in part in the following description, or can be learned from the description, or can be learned through practice of the example embodiments.

According to the proposed solution, a wearable computing device is provided. The wearable computing device includes a housing having an upper side and a lower side, where the lower side of the housing is opposite to the upper side of the housing and is configured to be in contact with a body part of a user when the wearable computing device is worn by the user. The wearable computing device also includes a user interface disposed on the upper side of the housing and sensors which are disposed on the lower side of the housing. The sensors are configured to output one or more optical readings when a force is applied to the user interface. The sensors include photoplethysmography (PPG) sensors configured to monitor a heart rate of the user when the wearable computing device is worn by the user, and the PPG sensors include one or more light-emitting diodes and a plurality of detectors. The wearable computing device further includes one or more processors configured to determine a location at which the force is applied to the user interface based on the one or more optical readings output by the PPG sensors.

In some implementations, the sensors may include at least three photoplethysmography (PPG) sensors, and at least one PPG sensor of the at least three PPG sensors is spaced apart from another PPG sensor of the at least three PPG sensors in a first direction and a second direction.

In some implementations, the one or more processors may be configured to execute one or more functions of the wearable computing device based on the location at which the force is applied to the display as determined by the one or more processors.

In some implementations, the one or more processors may be configured to determine the location at which the force is applied to the user interface based on whether an area of the user interface to which the force is applied corresponds to a first area or a second area, the first area being different from than the second area. For example, the first area may be larger than the second area.

In some implementations, when the area of the display to which the force is applied corresponds to the first area, the one or more processors may be configured to determine an amplitude of each of the one or more optical readings based on a first model having a first scale parameter which reflects a first variance of the force applied to the user interface, and when the area of the display to which the force is applied corresponds to the second area, the one or more processors may be configured to determine the amplitude of each of the one or more optical readings based on a second model having a second scale parameter which reflects a second variance of the force applied to the user interface, the second variance being different from the first variance.

In some implementations, the one or more processors may be configured to determine the location at which the force is applied to the user interface based on a value of each of the optical readings output by the sensors and by applying a loss function to minimize a difference between an actual location at which the force is applied and an estimated location at which the force is applied. In such an implementation, the one or more processors may be configured to determine the location at which the force is applied to the user interface using a value of each of the one or more optical readings output by the sensors and known (i.e., predefined and stored) positions of the sensors to estimate the location at which the force is applied by applying a loss function. An estimated location resulting from minimizing the loss function may then be used as the determined location at which the force is applied. In particular, determining the location at which the force is applied may therefore include using an estimate of the location resulting from applying a loss function, where the loss function includes values of each of the one or more optical readings and known positions of the sensors at the lower side of the wearable computing device's housing. The loss function may further include a probability density function determining a probability of an optical reading output by an optical sensor being corrupted by measurement noise. In some implementations the probability density function may be a standard Gaussian function which is used for reconstructing a location of the application of a force based on measurements from the one or more sensors.

In some implementations, the one or more processors may be configured to apply the loss function by performing a two-dimensional grid search method or a gradient descent search method.

In some implementations, the user interface includes a non-touch sensitive display configured to display a plurality of elements corresponding to a plurality of selectable functions of the wearable computing device. In response to a noise level of the sensors increasing beyond a threshold, the one or more processors may be configured to control the display to stop the display of one or more of the plurality of elements, and in response to determining the location at which the force is applied to the display corresponds to a remaining first element of the plurality of elements displayed on the display, the one or more processors may be further configured to execute a first selectable function of the plurality of selectable functions which corresponds to the remaining first element, based on the determined location.

In some implementations, the user interface includes a plurality of faux buttons which respectively correspond to a plurality of selectable functions of the wearable computing device, and in response to determining the location at which the force is applied to a first faux button of the plurality of faux buttons of the user interface, the one or more processors are configured to execute a first selectable function of the plurality of selectable functions which corresponds to the first faux button, based on the determined location.

In some implementations, the user interface includes a display, and the display is a non-touch sensitive display.

In some implementations, the sensors may include photoplethysmography (PPG) sensors configured to monitor a heart rate of the user when the wearable computing device is worn by the user, and the one or more processors may be configured to identify a gesture of the user based on the one or more optical readings output by the PPG sensors. The one or more processors may be configured to execute a function of the computer wearable device based on the gesture of the user identified by the one or more processors.

In some implementations, the wearable computing device may further include at least one of an accelerometer or a gyroscope, and the one or more processors may be configured to identify the gesture of the user based on the one or more optical readings output by the PPG sensors and one or more outputs of the at least one of the accelerometer or the gyroscope.

According to the proposed solution, a computer-implemented method is provided. The computer-implemented method includes receiving (e.g., by one or more processors of a wearable computing device) optical readings output by sensors disposed on a lower side of a housing of the wearable computing device, when a force is applied to a user interface disposed on an upper side of the housing. The lower side of the housing is opposite to the upper side of the housing and is configured to be in contact with a body part of a user when the wearable computing device is worn by the user. The sensors include photoplethysmography (PPG) sensors configured to monitor a heart rate of the user when the wearable computing device is worn by the user, and the PPG sensors include one or more light-emitting diodes and a plurality of detectors. The method further includes determining, by the one or more processors of the wearable computing device, a location at which the force is applied to the user interface based on the one or more received optical readings of the PPG sensors.

In some implementations, the method may further include executing one or more functions of the wearable computing device based on the location at which the force is applied to the user interface as determined by the one or more processors.

In some implementations, the method may further include displaying on a display of the user interface instructions indicating to a user to calibrate the wearable computing device by indicating an area of the display to which the force is to be applied to the display.

In some implementations, the method may further include, when the area of the force indicated by the user corresponds to a first area, determining an amplitude of each of the one or more optical readings based on a first model having a first scale parameter which reflects a first variance of the force to be applied to the display, and when the area of the force indicated by the user corresponds to a second area, determining the amplitude of each of the one or more optical readings based on a second model having a second scale parameter which reflects a second variance of the force to be applied to the display, the first area being greater than the second area, and the first variance being greater than the second variance.

In some implementations, the method may further include determining the location at which the force is applied to the user interface based on a value of each of the optical readings output by the sensors and by applying a loss function to minimize a difference between an actual location at which the force is applied and an estimated location at which the force is applied.

In some implementations, the method may further include displaying, on a non-touch sensitive display of the user interface, a plurality of elements corresponding to a plurality of selectable functions of the wearable computing device, and in response to a noise level of the sensors increasing beyond a threshold, controlling the display to stop the display of one or more of the plurality of elements, and in response to determining the location at which the force is applied to the user interface corresponds to a remaining first element of the plurality of elements displayed on the display, executing a first selectable function of the plurality of selectable functions which corresponds to the remaining first element, based on the determined location.

In an example embodiment, a non-transitory computer-readable medium which stores instructions that are executable by one or more processors of a wearable computing device is provided. The non-transitory computer-readable medium stores instructions including instructions to cause the one or more processors to receive one or more optical readings output by sensors disposed on a lower side of a housing of the wearable computing device, when a force is applied to a user interface disposed on an upper side of the housing, wherein the lower side of the housing is opposite to the upper side of the housing and configured to be in contact with a body part of a user when the wearable computing device is worn by the user. The sensors include photoplethysmography (PPG) sensors configured to monitor a heart rate of the user when the wearable computing device is worn by the user, and the PPG sensors include one or more light-emitting diodes and a plurality of detectors. The non-transitory computer-readable medium stores instructions including instructions to cause the one or more processors to determine a location at which the force is applied to the user interface based on the one or more received optical readings of the PPG sensors. The non-transitory computer-readable medium stores instructions including instructions to execute one or more functions of the wearable computing device based on the location at which the force is applied to the user interface as determined by the one or more processors. The non-transitory computer-readable medium may store additional instructions to execute other aspects of the wearable computing device and computer-implemented method as described herein.

These and other features, aspects, and advantages of various embodiments of the disclosure will become better understood with reference to the following description, drawings, and appended claims. The accompanying drawings, which are incorporated in and constitute a part of this specification, illustrate example embodiments of the disclosure and, together with the description, serve to explain the related principles.

### BRIEF DESCRIPTION OF THE DRAWINGS

Detailed discussion of example embodiments directed to one of ordinary skill in the art is set forth in the specification, which makes reference to the appended drawings, in which:
FIG. 1 depicts an example wearable computing device according to according to one or more example embodiments of the disclosure;
FIG. 2 depicts an exploded view of an example wearable computing device according to one or more example embodiments of the disclosure;
FIG. 3 depicts an example block diagram of the wearable computing device according to one or more example embodiments of the disclosure;
FIGS. 4A and 4B depict top views of example wearable computing devices according to one or more example embodiments of the disclosure;
FIG. 4C depicts a bottom view of an example wearable computing device according to one or more example embodiments of the disclosure;
FIGS. 5A and 5B depict a force applied to a display of the wearable computing device and a corresponding signal response diagram of sensors, according to one or more example embodiments of the disclosure;
FIGS. 6A and 6B depict example press Gaussian responses corresponding to different scale parameters, according to one or more example embodiments of the disclosure;
FIGS. 7A and 7B depict example observation values according to one or more example embodiments of the disclosure;
FIGS. 8A and 8B depict an example determination of the location of a force applied to the display based on a loss function result, according to one or more example embodiments of the disclosure;
FIGS. 9A and 9B depict another example determination of the location of a force applied to the display based on a loss function result, according to one or more example embodiments of the disclosure;
FIGS. 10A and 10B depict an example determination of the location of a force applied to the display based on a loss function result in a noisy environment, according to one or more example embodiments of the disclosure;
FIG. 11 depicts example response signals of various sensors of the wearable computing device to detect a gesture of a user, according to one or more example embodiments of the disclosure;
FIGS. 12A and 12B depict example one-handed gestures of a user and corresponding heatmaps reconstructed from PPG sensors, according to one or more example embodiments of the disclosure; and
FIGS. 13 and 14 each illustrate a flow diagram of an example, non-limiting computer-implemented method according to one or more example embodiments of the disclosure.

### DETAILED DESCRIPTION

Reference now will be made to embodiments of the disclosure, one or more examples of which are illustrated in the drawings. Each example is provided by way of explanation of the disclosure and is not intended to limit the disclosure.

Terms used herein are used to describe the example embodiments and are not intended to limit and / or restrict the disclosure. The singular forms "a," "an" and "the" are intended to include the plural forms as well, unless the context clearly indicates otherwise. In this disclosure, terms such as "including", "having", "comprising", and the like are used to specify features, numbers, steps, operations, elements, components, or combinations thereof, but do not preclude the presence or addition of one or more of the features, elements, steps, operations, elements, components, or combinations thereof.

It will be understood that, although the terms first, second, third, etc., may be used herein to describe various elements, the elements are not limited by these terms. Instead, these terms are used to distinguish one element from another element. For example, without departing from the scope of the disclosure, a first element may be termed as a second element, and a second element may be termed as a first element.

The term "and / or" includes a combination of a plurality of related listed items or any item of the plurality of related listed items. For example, the scope of the expression or phrase "A and/or B" includes the item "A", the item "B", and the combination of items "A and B".

In addition, the scope of the expression or phrase "at least one of A or B" is intended to include all of the following: (1) at least one of A, (2) at least one of B, and (3) at least one of A and at least one of B. Likewise, the scope of the expression or phrase "at least one of A, B, or C" is intended to include all of the following: (1) at least one of A, (2) at least one of B, (3) at least one of C, (4) at least one of A and at least one of B, (5) at least one of A and at least one of C, (6) at least one of B and at least one of C, and (7) at least one of A, at least one of B, and at least one of C.

Examples of the disclosure are directed to a wearable computing device that can be worn, for example, on a user's wrist. For example, the wearable computing device may include a watch. The wearable computing device includes a housing having an upper side and a lower side. The lower side of the housing is opposite to the upper side of the housing and is configured to be in contact with a body part of a user when the wearable computing device is worn by the user. The wearable computing device further includes a user interface disposed on the upper side of the housing and sensors disposed on the lower side of the housing. The sensors may be configured to output one or more optical readings when a force is applied to the user interface. The wearable computing device may further include one or more processors configured to determine a location at which the force is applied to the user interface based on the one or more optical readings output by the sensors and a loss function described herein. The user interface is a tangible medium by which a user interacts with the wearable computing device. In an example, a user interface which is disposed on the upper side of the housing may include, for example, a display (which may be non-touch sensitive), one or more faux buttons, or the combination of the display and one or more faux buttons.

In an example embodiment, the wearable computing device can include a housing with a display (which may be non-touch sensitive) on an upper side of the housing and sensors on a lower side of the housing. The lower side of the housing is opposite to the upper side of the housing and is configured to be in contact with a body part of a user when the wearable computing device is worn by the user. The sensors may be used to indirectly sense a location of a touch by a user on the display. For example, when a user presses the display, optical readings from the sensors may fluctuate based on where the user presses. Optical readings from a sensor which is located closer to the location where the user presses sense more of the optical back-reflections compared to another sensor which is located further away from the location where the user presses. For example, based on the optical readings obtained from the sensors, one or more processors can determine a location where the user touches on the display and a function of the wearable computing device corresponding to the determined location can be carried out. Determining the location may involve an estimate of location parameters, e.g., location coordinates, based on a loss function implemented with the optical readings.

As an example, the display may display on a graphical user interface of the display four different visual elements, each visual element corresponding to a separate function of the wearable computing device, in respective quadrants of the display. If a user applies a force to an upper left quadrant of the display, for example to "select" the function represented by the visual element displayed in that quadrant, the sensors may output respective optical readings in response to the applied force. For example, a first sensor which is located closest to a location at which the force is applied to the display may output a signal with a higher amplitude or higher value compared to a second sensor which is located further away from the location at which the force is applied to the display. The one or more processors may receive one or more optical readings from each of the sensors and determine a location at which the force is applied to the display based on the one or more optical readings output by the sensors. If the one or more processors determine that the force is applied to a location corresponding to the upper left quadrant of the display based on the received one or more optical readings, the one or more processors may execute the function corresponding to the visual element displayed in the upper left quadrant of the display.

The sensors can include, for example, multipath photoplethysmography (PPG) sensors which can also be used to monitor a heart rate of the user. The PPG sensors may include one or more emitters (e.g., light-emitting diodes (LEDs)) and a plurality of detectors (e.g., two or more photodiodes). The detectors may be spaced apart from one another at various locations on the lower side of the housing so as to increase the accuracy of estimating the location of the touch by the user on the display.

In an example, the sensors may include at least three PPG sensors, and at least one PPG sensor of the at least three PPG sensors is spaced apart from another PPG sensor of the at least three PPG sensors in a first direction (e.g., X-axis) and a second direction (e.g., Y-axis). That is, the at least three PPG sensors are not arranged in a linear manner.

The one or more processors may be configured to determine the location at which the force is applied to the display based on whether an area of the display to which the force is applied corresponds to a first area or a second area, where the first area is different from the second area. For example, if a user applies their thumb to the display, then the area of the display to which the force is applied is larger than if the user were to apply their index finger to the display. As another example, if a user has large fingers (e.g., relative to an average human finger size for a male and/or female), it can be considered that the area of the display to which the force is applied would be larger than a user having average size fingers. Accordingly, during a calibration of the wearable computing device a user may provide an input to the wearable computing device (e.g., via a mechanical button) indicating the size or area of the display which is to receive the force applied from the user. The input may indicate the area size (e.g., a first area or a second area), which can be used to determine a value for a scale parameter (e.g., a first scale parameter or a second scale parameter). The value for the scale parameter may indicate or describe a diffuseness of a Gaussian response used to analyze the optical signals output from the sensors for determining a location of the force applied to the display. As is known, a Gaussian response when graphed generally has a symmetric "bell curve" shape. For a given application of force to a particular location, a size of an area to which the force is applied affects the magnitude of the pressure and an amplitude of the force is greatest at a central location of the force. A distribution of the amplitude of the force (and correspondingly the pressure) declines (e.g., exponentially) at points located further away from the central location of the force. A larger area will have greater diffuseness than a smaller area.

For example, if the user applies a force to the display with a thumb, then a higher-variance response would be experienced, and a higher scale parameter may be appropriate (e.g., a value of 10). On the other hand, if a user applies a force to the display with an index finger, a lower-variance response would be experienced, and a lower scale parameter value may be appropriate (e.g., a value of 1).

The scale parameter values may be implemented in a model which describes the touch response amplitude of the sensors which is registered by the wearable computing device. For example, the touch response amplitude that is registered by the wearable computing device may be approximately modeled as a one-mode Gaussian function: f_press(x, y) = EXP { -[ (x, y) - (x_press, y_press) ]^2 / scale }.

Here, (x, y) represents the coordinate of the particular sensor (i.e., the coordinate of the particular detector which may be a photodiode) reading the PPG fluctuations. The term (x_press, y_press) represents the ground truth coordinates of the location at which a force is applied to the top of the wearable computing device. The scale parameter describes the "diffuseness" of the Gaussian response, as explained above, and is used in modeling the captured optical readings based on the Gaussian function for taking into account an area covered by a touch which is user specific or user-group specific.

For example, when the area of the user interface (e.g., a display and/or faux buttons) to which the force is applied corresponds to a first area (e.g., for a large finger size or for a thumb), the one or more processors may be configured to determine an amplitude of each of the one or more optical readings output by the sensors based on a first model having a first scale parameter (e.g., having a value of 10) which reflects or describes a first variance of the force applied to the user interface. Alternatively, or in addition, when the area of the user interface (e.g., a display and/or faux buttons) to which the force is applied corresponds to a first area, the one or more processors may be configured to utilize a first Gaussian model to determine a location at which the force is applied to the user interface using a first loss function having the first scale parameter (e.g., having the value of 10) which reflects or describes the first variance of the force applied to the user interface.

For example, when the area of the user interface (e.g., a display and/or faux buttons) to which the force is applied corresponds to a second area (e.g., for a normal or small finger size or for an index finger), the one or more processors may be configured to determine the amplitude of each of the one or more optical readings output by the sensors based on a second model having a second scale parameter (e.g., having a value of 1) which reflects a second variance of the force applied to the user interface. Alternatively, or in addition, when the area of the user interface (e.g., a display and/or faux buttons) to which the force is applied corresponds to the second area, the one or more processors may be configured to utilize a second Gaussian model to determine the location at which the force is applied to the user interface using a second loss function having the second scale parameter (e.g., having the value of 1) which reflects or describes the second variance of the force applied to the user interface. Here, the first variance is different from (e.g., greater than) the second variance.

Stated differently, the one or more processors may be configured to determine the location of the force applied to the user interface based on a first scale parameter when the force applied to the user interface corresponds to a first area of the user interface (e.g., corresponding to the size of a user's thumb), and the one or more processors may be configured to determine the location of the force applied to the user interface based on a second scale parameter when the force applied to the user interface corresponds to a second area of the user interface (e.g., corresponding to the size of a user's index finger). In this case the first scale parameter is greater than the second scale parameter, and the first area is greater than the second area.

According to an example of the disclosure, the one or more processors of the wearable computing device may receive a number of observations from the sensors at a given point in time which corresponds to the number of sensors. For example, if the wearable computing device has N PPG sensors (in particular N detectors), then the one or more processors of the wearable computing device may receive N observations from the detectors. $\begin{matrix}obs_ 1 = f_press \left(x_PPG_1,y_PPG_1\right) # PPG_ 1 measures this value \\ obs_ 2 = f_press \left(x_PPG_2,y_PPG_1\right) # PPG_ 2 measures this value \\ … \\ obs_N = f_press \left(x_PPG_N, y_PPG_N\right) # PPG_N measures this value\end{matrix}$

Thus, if the wearable computing device has 3 detectors, then the one or more processors of the wearable computing device may receive 3 observations (i.e., 1 observation from each of the detectors) at a given point in time.

According to some implementations, the closer the detector observation point of a sensor is from the center of the Gaussian response of the force applied to the display, then the sensor reads more PPG fluctuations, and an output value of the sensor may be higher (e.g. a value of 0.98). The further the PD is the output value may decrease (e.g., from 0.98 to 0.92).

For example, the one or more processors may be configured to determine the location at which the force is applied to the display based on an optical reading of a sensor among the sensors having a highest value, among the one or more optical readings output by the sensors. For example, the one or more processors may be configured to determine the location at which the force is applied to the display based on a highest amplitude among the amplitudes of the one or more optical readings output from the sensors. For example, the one or more processors may be configured to determine the location at which the force is applied to the display based on one or more of the amplitudes of the one or more optical readings output from the sensors and a loss function described herein.

Examples of the disclosure are also directed to a non-transitory computer-readable medium which stores instructions that are executable by one or more processors of a wearable computing device. The instructions may include instructions to cause the one or more processors to receive one or more optical readings output by sensors disposed on a lower side of a housing of the wearable computing device, when a force is applied to a display disposed on an upper side of the housing. The lower side of the housing may be opposite to the upper side of the housing and be configured to be in contact with a body part of a user when the wearable computing device is worn by the user. The instructions may further include instructions to cause the one or more processors to determine a location at which the force is applied to the display based on the one or more received optical readings.

The instructions may further include instructions to cause the one or more processors to execute one or more functions of the wearable computing device based on the location at which the force is applied to the display. Example functions of the wearable computing device may include checking health information about the user such as a blood pressure, making and/or receiving a phone call, sending and/or receiving a text message, obtaining a current time, setting a timer, a stopwatch function, controlling an external device such as a home appliance, and the like.

The instructions may further include instructions to cause the one or more processors to display a graphical user interface indicating to a user to calibrate the wearable computing device by indicating an area of the display to which a force is to be applied to the display. When the area of the force indicated by the user corresponds to a first area, the instructions further include instructions to cause the one or more processors to determine an amplitude of each of the one or more optical readings based on a first model having a first scale parameter which reflects a first variance of the force to be applied to the display. When the area of the force indicated by the user corresponds to a second area, the instructions further include instructions to cause the one or more processors to determine the amplitude of each of the one or more optical readings based on a second model having a second scale parameter which reflects a second variance of the force to be applied to the display. For example, the first area may be greater than the second area, and the first variance may be greater than the second variance. The instructions may further include instructions to cause the one or more processors to determine the location at which the force is applied to the display based on a highest amplitude among the amplitudes of the one or more optical readings.

The instructions may further include instructions to cause the one or more processors to determine the location at which the force is applied to the display based on an optical reading of a sensor among the sensors having a highest value, among the one or more optical readings received by the one or more processors.

Examples of the disclosure are also directed to computer implemented methods of a wearable computing device. The method may include receiving one or more optical readings output by sensors disposed on a lower side of a housing of the wearable computing device, when a force is applied to a display disposed on an upper side of the housing. The lower side of the housing may be opposite to the upper side of the housing and be configured to be in contact with a body part of a user when the wearable computing device is worn by the user. The method may further include determining, by one or more processors of the wearable computing device, a location at which the force is applied to the display based on the one or more received optical readings.

The method may further include executing one or more functions of the wearable computing device based on the location at which the force is applied to the display. Example functions of the wearable computing device may include checking health information about the user such as a blood pressure, making and/or receiving a phone call, sending and/or receiving a text message, obtaining a current time, setting a timer, a stopwatch function, controlling an external device such as a home appliance, and the like.

The method may further include displaying a graphical user interface indicating to a user to calibrate the wearable computing device by indicating an area of the display to which a force is to be applied to the display. When the area of the force indicated by the user corresponds to a first area, the method may include determining an amplitude of each of the one or more optical readings based on a first model having a first scale parameter which reflects a first variance of the force to be applied to the display. When the area of the force indicated by the user corresponds to a second area, the method may include determining the amplitude of each of the one or more optical readings based on a second model having a second scale parameter which reflects a second variance of the force to be applied to the display. For example, the first area may be greater than the second area, and the first variance may be greater than the second variance. The method may include determining the location at which the force is applied to the display based on a highest amplitude among the amplitudes of the one or more optical readings. The method may include determining the location at which the force is applied to the display based on one or more of the amplitudes of the one or more optical readings output from the sensors and a loss function described herein.

The method may include determining the location at which the force is applied to the display based on an optical reading of a sensor among the sensors having a highest value, among the one or more optical readings received by the one or more processors.

According to examples described herein, the one or more processors of the wearable computing device may determine the location of a user touching an element or part of the wearable computing device at an upper side by calculating an estimate of location parameters, e.g., location coordinates, based on a loss function that is optimized to find a ground truth coordinate location of where the force is applied to the upper side of the wearable computing device. In an example, the probabilistic channel given that an observation (for example, obs_1) could be corrupted by Gaussian measurement noise, may be written as: p(obs_1 | (x_press, y_press))= G[ EXP { -[ (x_1, y_1) - (x_press, y_press) ]^2 / scale k } - obs_1 ].

Here the G(.) function describes the probability density of a standard Gaussian function which may be used to reconstruct the location of the application of the force using measurements obtained from the detectors, which are sparsely provided and distributed. For example, a probability of receiving the values observed for each of the observations N is calculated based on candidate touch events (i.e., candidate locations where the force is estimated to be applied). Whatever candidate touch event maximizes the probability (or minimizes the loss) may be considered as the most likely location of the force (i.e., touch event). For example, an expression of the negative log likelihood of all the observations may be used to find the ground truth press coordinates, as follows: $\begin{matrix}LOSS = {\left\{EXP\left\{-{\left[\left(x_1,y_1\right)-\left(x_press, y_press\right)\right]}^{∧}2/scale k\right\}-obs_1\right\}}^{∧} 2 \\ + {\left\{EXP\left\{-{\left[\left(x_2,y_2\right)-\left(x_press, y_press\right)\right]}^{∧}2/scale k\right\}-obs_1\right\}}^{∧} 2 \\ … + \left\{EXP\left\{-{\left[\left(x_N, y_N\right)-\left(x_press, y_press\right)\right]}^{∧}2/scale k\right\}-{\left.obs_N\right\}}^{∧}2\right\}\end{matrix}$

In the above expression, the terms "x_1, y_1", "x_2, y_2", and "x_N, y_N" correspond to the location of each PPG detector, which is known. The terms "obs_1", "obs_2", and "obs_N" correspond to the observed values of the respective detectors. The term "scale k" is the scale parameter value which describes the diffuseness of the Gaussian response as described herein. The terms "x_press, y_press" corresponds to the value that is to be estimated (i.e., the X, Y coordinate value of the location of the force). For example, in a three channel PPG sensor, where there are three detectors, N may be equal to three.

Because the example loss function is variable up to two parameters of interest (x_press, y_press), which at the time of observation are unknowns, a two-dimensional grid search may be performed to estimate (x_press, y_press) by finding the global loss minimizer. For example, the one or more processors may be configured to execute a program to perform a direct search in which every X, Y coordinate pair (e.g., corresponding to the area of the display) is tested as candidate touch events to obtain a result which achieves the minimal loss. The candidate touch event achieving the lowest loss (i.e., a minimal error) between may correspond to the estimated touch location. As another example, the one or more processors may implement a known gradient descent method to obtain a result (X, Y coordinate) which obtains the minimal loss. The gradient descent search method may utilize random candidate touch events (i.e., random candidate X, Y coordinates) to obtain a result which achieves the minimal loss rather than a direct search report in which all of the candidate touch events are tested. The gradient descent search method may use a smaller search space and thus be less computationally expensive than the direct search approach, saving energy and time. The disclosure is not limited to a direct search method or gradient descent search method, and other methods for minimizing a loss function may also be implemented.

In one or more examples, the user interface may include a non-touch sensitive display which displays a plurality of selectable functions of the wearable computing device. In response to a noise level of the sensors increasing beyond a threshold (e.g., a signal-to-noise ratio being greater than 10dB), the one or more processors may be configured to control the display so that a number of the plurality of selectable functions displayed on the display may be reduced, and the one or more processors may be configured to execute a function among the plurality of selectable functions based on the location at which the one or more processors determines the force is applied to the display. For example, a noise level of the sensors may increase when the wearable computing device is not securely held to the body part of the user and/or due to a low battery condition, such that the one or more optical readings may be affected by noise, and the accuracy of the sensors may be reduced. A number of the plurality of selectable functions displayed on the display may be reduced (e.g., reducing icons displayed on the display from ten icons to four icons) when the noise level of the sensors exceeds a threshold (e.g., a signal-to-noise ratio being greater than 10dB). Stopping the displaying of one or more of the plurality of selectable functions (i.e., reducing the number of the plurality of selectable functions displayed on the display) may enlarge a space on the display for each of the remaining selectable functions to be displayed. Thus, a likelihood that the one or more processors may accurately execute a function that the user intends to select may be increased, even though an accuracy of the detection or determination of the location of the force applied to the display may be reduced due to the noise encountered or experienced by the sensors. By changing the number of selectable functions displayed on the display, the noisy condition encountered by the sensors may be masked.

In one or more examples, the user interface may additionally, or alternatively, include a plurality of faux buttons which respectively correspond to a plurality of selectable functions of the wearable computing device. The one or more processors may be configured to execute a function of the computer wearable device among the plurality of selectable functions based on the location at which the force is applied with respect to respective locations of the plurality of faux buttons. For example, the faux buttons (or faux switch) may represent or indicate functions of the wearable computing device that can be performed by the wearable computing device. However, the faux buttons may not be mechanically or electrically connected (e.g., via a switch or capacitive sensors) to implement a function of the wearable computing device. Instead, the one or more processors may be configured to execute a function of the computer wearable device among the plurality of selectable functions based on the location at which the force is applied with respect to respective locations of the plurality of faux buttons.

In one or more examples, the wearable computing device may further include an accelerometer and/or a gyroscope to detect or sense motion data (e.g., for counting steps of a user) of the wearable computing device. The sensors may include PPG sensors which are also configured to monitor a heart rate of the user of the wearable computing device.

Motion of a user's hand affects arterial pressure and volumetric structure (muscle/tendon), creating a signature dip in reflection/absorption reflected in the PPG signal. The PPG sensors may capture the arterial pressure profiles spatially over a two-dimensional area (e.g., in x and y directions) on the lower side of the housing of the wearable computing device. The one or more processors may be configured to identify a gesture of the user based on the one or more optical readings output by the PPG sensors, and the one or more processors may be configured to execute a function of the computer wearable device (or another device) based on the gesture of the user identified by the one or more processors. The one or more processors may be configured to identify the gesture of the user based on the one or more optical readings output by the PPG sensors together with one or more outputs of the accelerometer and/or the gyroscope.

In one or more examples, the gesture of the user may include hand gestures including clinching of a fist, a pinching gesture with the fingers, squeezing of a hand, and the like. The gestures may be mapped to respective functions of the wearable computing device (e.g., to make a phone call), or a function of another device (e.g., to operate a home appliance) which may be controllable via the wearable computing device by the identification of the gesture. In one or more examples, the wearable computing device may identify a gesture based on the one or more optical readings output by the PPG sensors. For example, each gesture may correspond to a respective optical signal output by the PPG sensors so that a gesture may be correctly identified. In an example, optical signals output by the PPG sensors may be analyzed to identify the gesture (e.g., via machine learning algorithms to classify the output optical readings, by comparing the optical signals with known optical signals corresponding to respective gestures, etc.). To more accurately analyze the optical signals, portions of the optical readings which are attributed to a heart rate of the user may be filtered out. For example, a high pass heart rate filtering operation may be performed with respect to the optical readings to notch out the heart rate (arterial pulse), so that the majority of the optical readings which are used to identify the gesture corresponds to or is attributed to, the movement of the user's body part (e.g., the hand).

Example of the disclosure provide several technical effects, benefits, and/or improvements in computing technology and the technology of wearable computing devices. For example, according to one or more examples of the disclosure, a location of a touch on a display may be indirectly sensed by sensors disposed on a lower side of a housing of the wearable computing device which is in contact with a body part of a user when the wearable computing device is worn by the user. Accordingly, the wearable computing device need not have a touch-sensitive display, thereby reducing the number of parts included in the wearable computing device. Furthermore, according to one or more examples of the disclosure the wearable computing device leverages sensors which may be pre-existing in the wearable computing device. For example, the pre-existing sensors may include PPG sensors used for other purposes, such as detecting a heart rate of a user. Therefore, it is not necessary to add further sensors to the wearable computing device to determine a location of a touch by a user on the display. As another example, according to one or more examples of the disclosure the wearable computing device may utilize less power than a wearable computing device which includes a touch-sensitive display. As another example, according to one or more examples of the disclosure the wearable computing device may utilize less space than a wearable computing device which includes a touch-sensitive display. Therefore, the wearable computing device may be smaller and sleeker, or may provide space for other components which might not otherwise be installable if a touch-sensitive display were provided in the wearable computing device. As another example, according to one or more examples of the disclosure the wearable computing device may not include a display at all, and functions of the wearable computing device may be implemented using faux buttons which are associated with corresponding functions of the wearable computing device. Alternatively, according to one or more examples of the disclosure the wearable computing device may include a display as well as faux buttons, where functions of the wearable computing device may be implemented using the display and faux buttons, or only the faux buttons which are associated with corresponding functions of the wearable computing device.

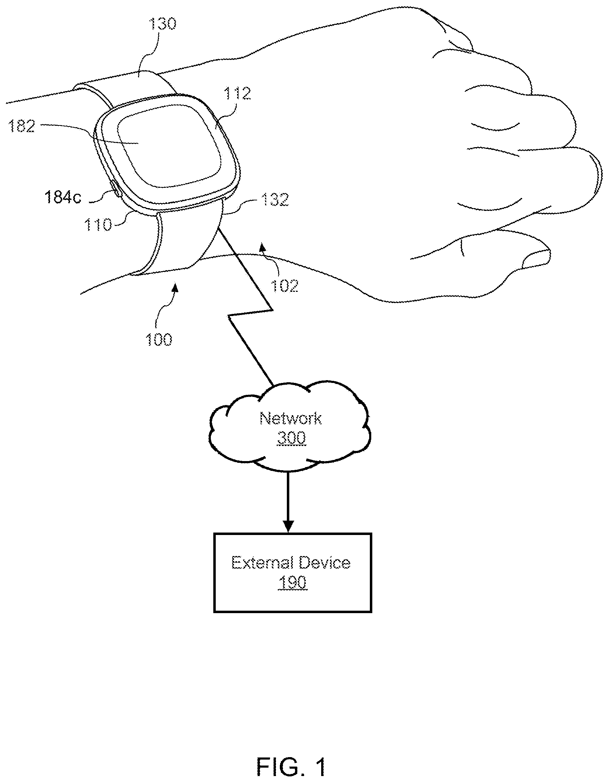

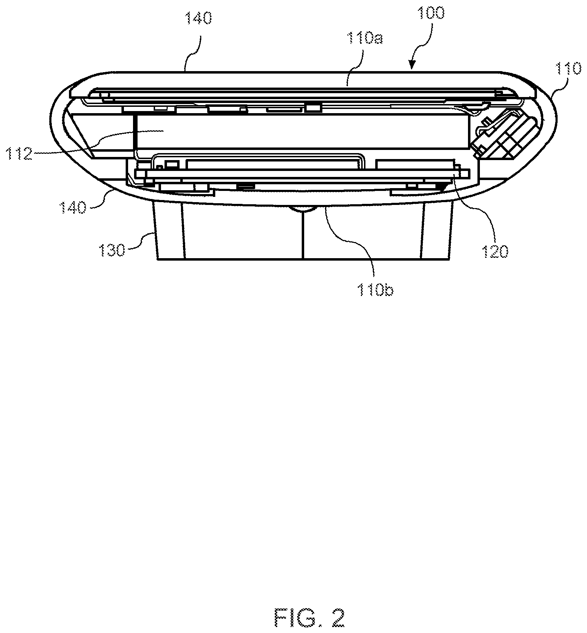

Referring now to the drawings, FIGS. 1 through 4B illustrate examples of a wearable computing device 100 according to examples of the disclosure. FIG. 1 illustrates an example wearable computing device 100 which can be worn, for example, on an arm 102 (e.g., wrist) of a user. For example, the wearable computing device 100 can include a housing 110 (e.g., a body). FIG. 2 illustrates an exploded view of the wearable computing device 100 where the housing 110 includes an upper side 110a and a lower side 110b, and the housing 110 can define a cavity 112 in which one or more electronic components (e.g., disposed on one or more printed circuit boards) are disposed. For example, the wearable computing device 100 can include a printed circuit board 120 disposed within the cavity 112. Furthermore, one or more electronic components can be disposed on the printed circuit board 120. The wearable computing device 100 can further include a battery (not shown) that is disposed within the cavity 112 defined by the housing 110. The wearable computing device 100 can further include various sensors 170 that are disposed within the cavity 112 defined by the housing 110. For example, the sensors 170 may include multipath photoplethysmography (PPG) sensors 172 disposed at the lower side 110b of the housing 110 which may be used to monitor a heart rate of the user. The PPG sensors 172 may include one or more emitters (e.g., light-emitting diodes (LEDs)) and a plurality of detectors (e.g., photodiodes). Light emitted from the one or more emitters is transmitted in a direction toward the user's body part (e.g., a portion of a user's wrist) which is in contact with the lower side 110b of the housing 110. The light then interacts with blood vessels of the user, where it is modified to a degree that is influenced by the current blood volume in the blood vessels. The modified light is directed back toward the PPG detectors by reflection and/or refraction. The PPG detectors generate data (e.g., one or more signals) which is reflective of the current blood volume of the blood vessels of the user which received the light emitted from the one or more emitters. For example, the sensors 170 may also include an accelerometer 174 which may be used to capture motion information with respect to the wearable computing device 100. For example, the sensors 170 may also include a gyroscope 176 which may also be used to capture motion information with respect to the wearable computing device 100.

The wearable computing device 100 can include a first band 130 and a second band 132. As shown, the first band 130 can be coupled to the housing 110 at a first location thereon. Conversely, the second band 132 can be coupled to the housing 110 at a second location thereon. Furthermore, the first band 130 and the second band 132 can be coupled to one another to secure the housing 110 to the arm 102 of the user.

In some examples, the first band 130 can include a buckle or clasp (not shown). Additionally, the second band 132 can include a plurality of apertures (not shown) spaced apart from one another along a length of the second band 132. In such implementations, a prong of the buckle associated with the first band 130 can extend through one of the plurality of openings defined by the second band 132 to couple the first band 130 to the second band 132. It should be appreciated that the first band 130 can be coupled to the second band 132 using any suitable type of fastener. For example, in some implementations, the first band 130 and the second band 132 can include a magnet. In such implementations, the first band 130 and the second band 132 can be magnetically coupled to one another to secure the housing 110 to the arm 102 of the user.

The wearable computing device 100 can include a cover 140 positioned on the housing 110 so that the cover 140 is positioned on top of (over) the display 182. In this manner, the cover 140 can protect the display 182 from being scratched. In some implementations, the wearable computing device 100 can include a seal (not shown) positioned between the housing 110 and the cover 140. For instance, a first surface of the seal can contact the housing 110 and a second surface of the seal can contact the cover 140. In this manner, the seal between the housing 110 and the cover 140 can prevent a liquid (e.g., water) from entering the cavity 112 defined by the housing 110.

It should be understood that the cover 140 can be optically transparent so that the user can view information being displayed on the display 182. For instance, in some implementations, the cover 140 can include a glass material. It should be understood, however, that the cover 140 can include any suitable optically transparent material.

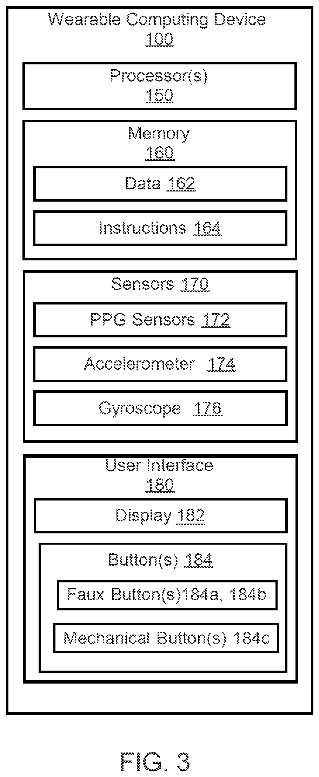

FIG. 3 illustrates an example block diagram of the wearable computing device 100 according to one or more example embodiments of the disclosure. The wearable computing device 100 may include one or more processors 150, one or more memory devices 160, one or more sensors 170, and a user interface 180.

For example, the one or more processors 150 can be any suitable processing device that can be included in a wearable computing device 100. For example, such a processor 150 may include one or more of a processor, processor cores, a controller and an arithmetic logic unit, a central processing unit (CPU), a graphics processing unit (GPU), a digital signal processor (DSP), an image processor, a microcomputer, a field programmable array, a programmable logic unit, an application-specific integrated circuit (ASIC), a microprocessor, a microcontroller, etc., and combinations thereof, including any other device capable of responding to and executing instructions in a defined manner. The one or more processors 150 can be a single processor or a plurality of processors that are operatively connected, for example in parallel.

The memory 160 can include one or more non-transitory computer-readable storage mediums, such as such as a Read Only Memory (ROM), Programmable Read Only Memory (PROM), Erasable Programmable Read Only Memory (EPROM), and flash memory, a USB drive, a volatile memory device such as a Random Access Memory (RAM), a hard disk, floppy disks, a blue-ray disk, or optical media such as CD ROM discs and DVDs, and combinations thereof. However, examples of the memory 160 are not limited to the above description, and the memory 160 may be realized by other various devices and structures as would be understood by those skilled in the art.

For example, memory 160 can store instructions, that when executed, cause the one or more processors 150 to determine a location at which a force is applied to the display 182 based on one or more received optical readings output by PPG sensors 172, as described according to examples of the disclosure. For example, memory 160 can store instructions, that when executed, cause the one or more processors 150 to execute one or more functions of the wearable computing device 100 based on the determined location, as described according to examples of the disclosure.

Memory 160 can also include data 162 and instructions 164 that can be retrieved, manipulated, created, or stored by the one or more processor(s) 150. In some example embodiments, such data can be accessed and used as input to determine a location at which a force is applied to the display 182 based on one or more optical readings (signals) output by the PPG sensors 172. In some examples, the memory 160 can include data used to perform one or more processes and instructions that execute one or more functions of the wearable computing device 100 based on the location at which the force is applied to the display 182 as determined by the one or more processors.

The wearable computing device 100 can include a user interface 180 configured to receive an input from a user by the user applying a force to the user interface (e.g., via a thumb, finger, or an input device such as a stylus or pen). The wearable computing device 100 may execute a function in response to receiving the input from the user (e.g., checking health information about the user such as a blood pressure, making and/or receiving a phone call, sending and/or receiving a text message, obtaining a current time, setting a timer, a stopwatch function, controlling an external device such as a home appliance, and the like).

For example, the wearable computing device 100 may be connected to one or more external devices 190 in a wireless and/or wired manner. The wearable computing device 100 may be connected to the external device 190 over a network 300 such as a local area network (LAN), wireless local area network (WLAN), wide area network (WAN), personal area network (PAN), virtual private network (VPN), or the like. For example, wireless communication between the wearable computing device 100 and the external device 190 may be performed via a wireless LAN, Wi-Fi, Bluetooth, ZigBee, Wi-Fi direct (WFD), ultra wideband (UWB), infrared data association (IrDA), Bluetooth low energy (BLE), near field communication (NFC), a radio frequency (RF) signal, and the like. For example, the wired communication connection may be performed via a USB cable, a pair cable, a coaxial cable, an optical fiber cable, an Ethernet cable, and the like.

The user interface 180 may include a display 182 which displays information viewable by the user (e.g., time, date, biometric information, notifications, etc.). For example, the display 182 may be a non-touch sensitive display. The display 182 may include a liquid crystal display (LCD), a light emitting diode (LED) display, an organic light emitting diode (OLED) display, active matrix organic light emitting diode (AMOLED), flexible display, 3D display, a plasma display panel (PDP), a cathode ray tube (CRT) display, and the like, for example. However, the disclosure is not limited to these example displays and may include other types of displays. That is, the non-touch sensitive display 182 may not be capable of sensing a touch event via conduction using electrical conductors (e.g., like a capacitive touch display or resistive display) to change a capacitance or resistance of a circuit. Thus, the non-touch sensitive display 182 does not include a touch panel which may include conductive layers and/or resistive layers of circuitry. The display 182 may include various layers, including one or more of a display surface, polarizing layers, liquid crystal layer, backlight, glass substrate, and the like. In some implementations, the display 182 may have a square or rectangular shape, or may be annular in shape (e.g., elliptical, circular, etc.). However, it should be appreciated that the display 182 can have any suitable shape.

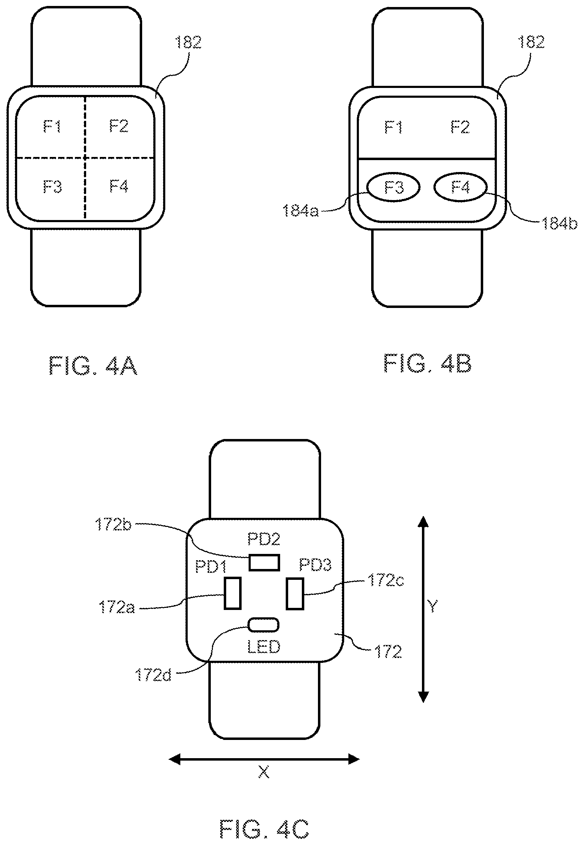

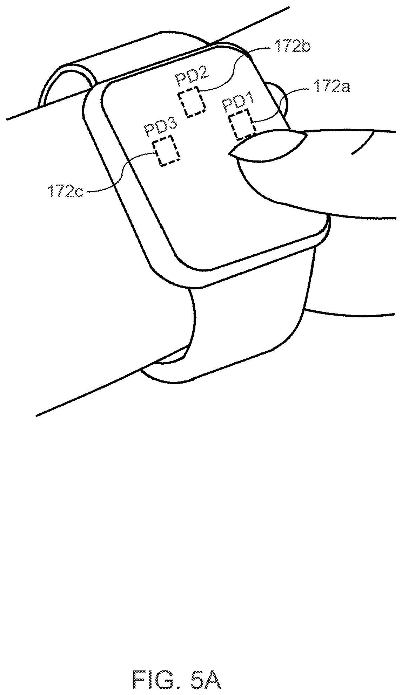

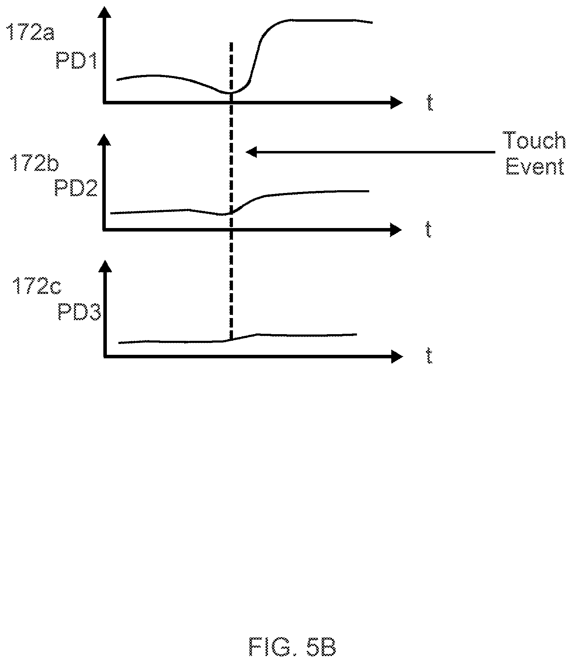

In the example of FIG. 4A, the display 182 displays various functions F1, F2, F3, F4 in respective quadrants of the display 182 that the wearable computing device 100 is capable of performing. For example, the functions may be displayed using visual elements such as text and/or by using symbols (e.g., icons) which inform the user of the respective function to be performed. For example, in response to a user applying a force to the lower right quadrant of the display 182 where the function F4 is represented, PPG detectors 172a, 172b, 172c may output respective optical readings (signals) S1, S2, S3 (see FIGS. 4C and 5A-5B) in response to the applied force. For example, a first PPG detector 172a which is located closest to the location at which the force is applied to the display 182 may output a signal S1 with a higher amplitude or higher value compared to the outputs S2 and S3 of second and third PPG detectors 172b, 172c which are located further away from the location at which the force is applied to the display 182. One or more processors 150 may receive one or more optical readings (signals) from each of the PPG detectors 172a, 172b, 172c and determine a location at which the force is applied to the display 182 based on the one or more optical readings (signals) output by the PPG detectors 172a, 172b, 172c. In response to the one or more processors 150 determining that the force is applied to a location corresponding to the lower right quadrant of the display 182 based on the received one or more optical readings (signals), the one or more processors 150 may execute the function F4 which is displayed in the lower right quadrant of the display 182.

The user interface 180 may additionally, or alternatively, include one or more buttons 184 to receive an input from a user by the user applying a force to the one or more buttons 184. For example, one or more buttons 184c may be included on one or more peripheral sides of the wearable computing device 100 as shown in FIG. 1, for example. The one or more buttons 184c may be a known button which includes mechanical components and/or electrical circuitry to implement a function of the wearable computing device 100 (e.g., setting a time, changing a setting and/or view of the display 182, selecting an option displayed on the display 182). In addition, or alternatively, the one or more buttons 184 may also include one or more faux buttons 184a, 184b. For example, in FIG. 4B, faux buttons 184a, 184b are disposed on an upper side 110a of the housing 110 adjacent to the display 182. The faux buttons 184a, 184b may represent or indicate functions F3, F4 of the wearable computing device 100 that can be performed by the wearable computing device 100. For example, the faux button 184a may include a label, symbol, or other indication to inform the user of the function F3 to be performed, and the faux button 184b may include a label, symbol, or other indication to inform the user of the function F4 to be performed. In another example, the wearable computing device 100 may not include the display 182 and the upper side of the housing 110 may include one or more faux buttons 184a, 184b to receive a user input. The faux buttons 184a, 184b may not be mechanically and/or electrically connected (e.g., via a switch or capacitive sensors) to other components of the wearable computing device 100 to implement a function of the wearable computing device 100.

For example, in response to a user applying a force to the faux button 184b, PPG detectors 172a, 172b, 172c may output respective optical readings (signals) S1, S2, S3 (see FIGS. 4C and 5A-5B) in response to the applied force. For example, a first PPG detector 172a which is located closest to the location at which the force is applied to the faux button 184b may output a signal S1 with a higher amplitude or higher value compared to the outputs S2 and S3 of second and third PPG detectors 172b, 172c which are located further away from the location at which the force is applied to the faux button 184b. One or more processors 150 may receive one or more optical readings (signals) from each of the PPG detectors 172a, 172b, 172c and determine a location at which the force is applied to the upper side of the housing 110 based on the one or more optical readings (signals) output by the PPG detectors 172a, 172b, 172c. In response to the one or more processors 150 determining that the force is applied to a location corresponding to the faux button 184b based on the received one or more optical readings (signals), the one or more processors 150 may execute the function F4 which is represented by faux button 184b.

With reference to FIG. 4C, a bottom view of an example wearable computing device is illustrated according to one or more example embodiments of the disclosure. The wearable computing device 100 may include a plurality of PPG sensors, for example to assist in rejecting motion artifacts. Each PPG sensor may correspond to a combination of a light source and a respective detector. For example, in FIG. 4C there are three PPG sensors: the combination of LED 172d with detector 172a, the combination of LED 172d with detector 172b, and the combination of LED 172d with detector 172c. However, the disclosure is not limited to the example of FIG. 4C and the wearable computing device 100 may include two PPG sensors or more than three PPG sensors. Furthermore, more than one LED may be included such that different detectors may be combined with different LEDs and/or each detector may be combined with one or more LEDs to output a PPG signal. For example, the plurality of detectors may be disposed in a circular or elliptical arrangement, where the plurality of detectors may be spaced apart from each other at regular or irregular intervals. In FIG. 4C, detector 172a is spaced apart from detector 172c in the horizontal or "X" direction, and is spaced apart from detector 172b in both the horizontal (X) direction and the vertical or "Y" direction. Detector 172b is spaced apart from both detectors 172a and 172c in the horizontal (X) direction and vertical (Y) direction. Detector 172c is spaced apart from detector 172a in the horizontal (X) direction, and is spaced apart from detector 172b in both the horizontal (X) direction and the vertical (Y) direction. LED 172d is spaced apart from detector 172b in the vertical (Y) direction and is spaced apart from both detectors 172a and 172c in each of the horizontal (X) and vertical (Y) directions. However, the configuration of the detectors and LED may be different from that illustrated in FIG. 4C, and the disclosure is not limited to the example of FIG. 4C.

The PPG sensors may optically measure biometric information such as a heart rate, using a light source and a photodetector at the surface of a user's body part to measure the volumetric variations of blood circulation. The light source (e.g., one or more LEDs) emits light to a body part and the photodetector measures the reflected light from the body part, where the amount of reflected light may indicate biometric information about the user (e.g., blood flow, volume of blood, etc.). The light source may emit infrared light, and the color of the LED may be red, green, or yellow. The PPG signal may include various components, such as a DC component (or DC offset), which represents the constant absorption of light passing through the tissues, and an AC component generated by heartbeats (cardiac activity) affecting blood volume, which depends on the systolic and diastolic phases.

As disclosed herein, PPG signals output by the PPG sensors may also be affected when a force (e.g., a direct or indirect force) is applied to a body part of the user where the PPG sensors are located. The changes in the PPG signals due to this force may be used to detect an event (e.g., a touch event on the display 182 when a force is applied to the display 182 of the wearable computing device 100).

For example, when a user applies a force (e.g., a pressing action) to an upper side 110a of the wearable computing device 100 (e.g., the user interface 180 including the display 182 and/or faux buttons 184a, 184b), optical readings (signals) from the PPG sensors 172 disposed on a lower surface of the wearable computing device 100 fluctuate based on where the user is applying the force. For example, a detector of the PPG sensor which is closest to the location of the force application will sense more of the optical back-reflections compared to another detector which is located further away from the location of the force application. The one or more processors may be configured to determine or estimate (e.g., using a sparse-pixel maximum likelihood decoder) the location at which the force is applied to the display (e.g., an X, Y position on the user interface 180 including the display 182 and/or faux buttons 184a, 184b).

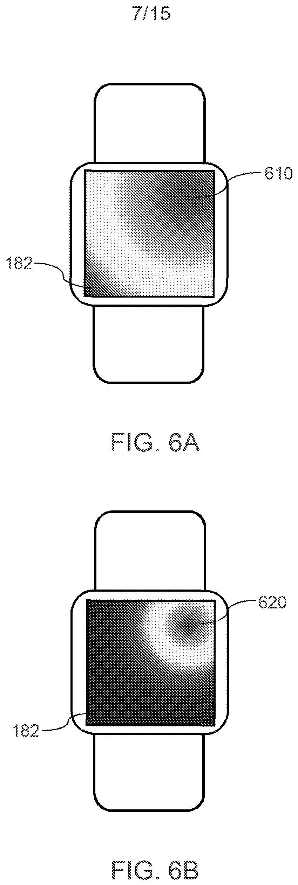

For example, a touch response amplitude of a detector that is output when a force is applied to the upper part of the wearable computing device 100 (e.g., the display 182) may be approximately modeled as a one-mode Gaussian function: f_press(x, y) = EXP { - [ (x, y) - (x_press, y_press) ]^2 / scale k }. Here, (x, y) represents the coordinate of the particular detector (e.g., photodiode) reading the PPG fluctuations. The term (x_press, y_press) represents the ground truth coordinates of the location at which a force is applied to the upper part of the wearable computing device. The scale parameter k describes the "diffuseness" of the Gaussian response, which is explained in more detail with reference to FIGS. 6A-10B.

For example, generally for a given application of a force to a particular location, an amplitude of the force is greatest at a central location of the force, and a distribution of the amplitude of the force declines (e.g., exponentially) at points located further away from the central location of the force. In modeling the Gaussian response, a scale parameter k may be determined based on whether an area of the display to which the force is applied corresponds to a first area or a second area, where the first area is larger than the second area. For example, if a user applies their thumb to the display, then the area of the display to which the force is applied is larger than if the user were to apply their index finger to the display. As another example, if a user has large fingers (e.g., relative to an average human finger size for a male and/or female), it can be considered that the area of the display to which the force is applied would be larger than a user having average size fingers.

Accordingly, during a calibration of the wearable computing device a user may provide an input to the wearable computing device indicating the size or area of the display which is to receive the force applied from the user. The input may indicate the area size (e.g., a first area or a second area), which can be used to determine a scale parameter (e.g., a first scale parameter k1 or a second scale parameter k2). The scale parameter may indicate or describe a diffuseness of the Gaussian response which can be used to analyze the optical signals output from the PPG sensors 172 for determining a location of the force applied to the display 182.

FIGS. 6A and 6B depict example press Gaussian responses corresponding to different scale parameters, according to one or more example embodiments of the disclosure For example, if the user applies a force to the display 182 with a thumb, then a higher-variance response 610 would be experienced, and a higher scale parameter k1 may be appropriate (e.g., a value of 10). On the other hand, if a user applies a force to the display 182 with an index finger, a lower-variance response 620 would be experienced, and a lower scale parameter value k2 may be appropriate (e.g., a value of 1).

The scale parameter values (e.g., k1 and k2) may be implemented in the model described above (e.g., using the Gaussian function f_press(x, y) = EXP { -[ (x, y) - (x_press, y_press) ]^2 / scale k) which describes the touch response amplitude of the PPG sensors 172 which is registered by the wearable computing device 100.

For example, when the area of the display 182 to which the force is applied corresponds to a first area (e.g., for a large finger size or for a thumb), the one or more processors 150 may be configured to determine an amplitude of each of the one or more optical readings output by the PPG sensors 172 based on a first model having a first scale parameter k1 (e.g., having a value of 10) which reflects or describes a first variance of the force applied to the display 182.

For example, when the area of the display 182 to which the force is applied corresponds to a second area (e.g., for a normal or small finger size or for an index finger), the one or more processors 150 may be configured to determine the amplitude of each of the one or more optical readings output by the PPG sensors 172 based on a second model having a second scale parameter k2 (e.g., having a value of 1) which reflects a second variance of the force applied to the display 182. Here, the first variance is greater than the second variance.

Stated differently, the one or more processors 150 may be configured to determine the location of the force applied to the display 182 based on a first scale parameter k1 when the force applied to the display 182 corresponds to a first area of the display 182 (e.g., corresponding to the size or surface area of a user's thumb), and the one or more processors 150 may be configured to determine the location of the force applied to the display 182 based on a second scale parameter k2 when the force applied to the display 182 corresponds to a second area of the display 182 (e.g., corresponding to the size or surface area of a user's index finger). In this case the first scale parameter k1 is greater than the second scale parameter k2, and the first area is greater than the second area. For example, the first area and second area may correspond to known or empirically obtained values for average fingertip areas of a thumb or index finger, and may additionally be categorized according to a gender of the user. As an example, a first area may correspond to about 5 cm² to 6 cm² (an approximate surface area of the tip of a thumb) and a second area may correspond to about 2.5 cm² to 3.5 cm² (an approximate surface area of the tip of an index finger).

For example, during a calibration of the wearable computing device 100 a user may provide an input to the wearable computing device 100 indicating the size or area of the display 182 which is to receive a force applied from the user. For example, the wearable computing device 100 may display information on the display 182 which requests that the user indicate a preferred digit (e.g., a thumb or index finger) to be used for providing an input (force) to the display 182. For example, the user may select the preferred digit using a button 184c disposed on an outer peripheral portion of the wearable computing device 100 similar to a manner in which a user may set a time or date as in known watches. The user may additionally and/or alternatively input their gender for further determination of the scale parameter k for selecting an appropriate model to be utilized.

According to examples of the disclosure, the one or more processors 150 of the wearable computing device 100 may receive a number of observations from the PPG sensors 172 at a given point in time which corresponds to the number of sensors. For example, if the wearable computing device has N PPG sensors 172 (in particular N detectors 172a-c), then the one or more processors 150 of the wearable computing device 100 may receive N observations from the detectors 172a-172c. $\begin{matrix}obs_ 1 = f_press \left(x_PPG_1,y_PPG_1\right) # PPG_ 1 measures this value \\ obs_ 2 = f_press \left(x_PPG_2,y_PPG_1\right) # PPG_ 2 measures this value \\ … \\ obs_N = f_press \left(x_PPG_N, y_PPG_N\right) # PPG_N measures this value\end{matrix}$

As illustrated in FIG. 4C, the wearable computing device 100 has three detectors 172a-172c. The one or more processors 150 of the wearable computing device 100 may receive three observations (i.e., one observation from each of the detectors 172a-172c) at a given point in time.

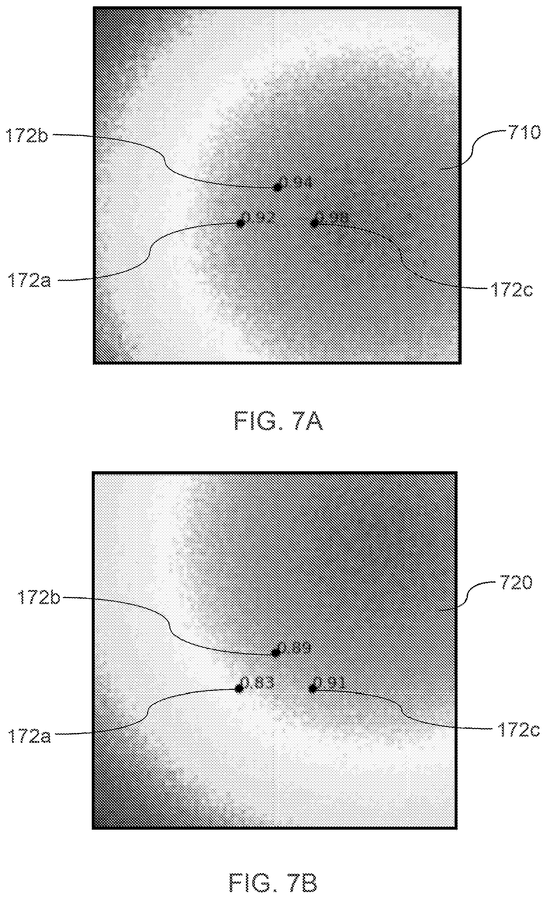

FIGS. 7A and 7B depict example observation values according to one or more example embodiments of the disclosure. As described previously, according to some implementations, the closer the detector observation point of a detector is from the center of the Gaussian response of the force applied to the display 182, then the detector reads more PPG fluctuations, and an output value of the detector may be higher while detectors located further away have lower values. For example, in FIG. 7A, outputs of the detectors 172a-172c are overlaid on top of a press Gaussian function 710, where darker shading indicates the center of the Gaussian function corresponding to the actual touch location. detector 172c outputs a value of 0.98 while detectors 172a and 172b output values of 0.92 and 0.94 respectively. The one or more processors 150 may determine or estimate that the location of the force applied to the display 182 is in a middle right portion of the display 182 based on the values of each of the detectors 172a-172c. For example, in FIG. 7B, detector 172c outputs a value of 0.91 while detectors 172a and 172b output values of 0.83 and 0.89 respectively. The one or more processors 150 may determine or estimate that the location of the force applied to the display 182 is in an upper right portion of the display 182, based on the values of each of the detectors 172a-172c, noting that detectors 172b and 172c have relatively similar values compared to the value output by detector 172a.

The one or more processors 150 may be configured to determine the location at which the force is applied to the display 182 based on optical reading values of a PPG sensor 172 among the PPG sensors 172 having a highest value, e.g., a highest voltage value. For example, the one or more processors 150 may be configured to determine the location at which the force is applied to the display 182 based on a highest amplitude among the amplitudes of the one or more optical reading values output from the PPG sensors 172.

The one or more processors 150 may be configured to determine the location at which the force is applied to the display 182 based on a loss function that is optimized to find a ground truth coordinate location of where the force is applied to the display 182. In an example, the probabilistic channel given that an observation (for example, obs_1) could be corrupted by Gaussian measurement noise, may be written as: p(obs_1 | (x_press, y_press))= G[ EXP { -[ (x_1, y_1) - (x_press, y_press) ]^2 / scale k } - obs_1 ].

Here the G(.) function describes the probability density of a standard Gaussian function which may be used to reconstruct the location of the application of the force using measurements obtained from the detectors, which are sparsely provided and distributed. For example, a probability of receiving the values observed for each of the observations N is calculated based on candidate touch events (i.e., candidate locations where the force is estimated to be applied). Whatever candidate touch event maximizes the probability (or minimizes the loss) may be considered as the most likely location of the force (i.e., touch event). For example, an expression of the negative log likelihood of all the observations may be used to find the ground truth press coordinates, as follows: $\begin{matrix}LOSS = {\left\{EXP\left\{-{\left[\left(x_1,y_1\right)-\left(x_press, y_press\right)\right]}^{∧}2/scale k\right\}-obs_1\right\}}^{∧} 2 \\ + {\left\{EXP\left\{-{\left[\left(x_2,y_2\right)-\left(x_press, y_press\right)\right]}^{∧}2/scale k\right\}-obs_1\right\}}^{∧} 2 \\ … + \left\{EXP\left\{-{\left[\left(x_N, y_N\right)-\left(x_press, y_press\right)\right]}^{∧}2/scale k\right\}-{\left.obs_N\right\}}^{∧}2\right\}\end{matrix}$

In the above expression, the terms "x_1, y_1", "x_2, y_2", and "x _N, y_N" correspond to the location of each PPG detector, which is known. The terms "obs_1", "obs_2", and "obs_N" correspond to the observed values of the respective detectors. The term "scale k" is the scale parameter value which describes the diffuseness of the Gaussian response as described herein. The terms "x_press, y_press" corresponds to the value that is to be estimated (i.e., the X, Y coordinate value of the location of the force). For example, in a three channel PPG sensor, where there are three detectors, N may be equal to three.

Because the example loss function is variable up to two parameters of interest (x_press, y_press), which at the time of observation are unknowns, a two-dimensional grid search may be performed to estimate (x_press, y_press) by finding the global loss minimizer. For example, the one or more processors 150 may be configured to execute a program to perform a direct search in which every X, Y coordinate pair (e.g., corresponding to the area of the display) is tested as candidate touch events to obtain a result which achieves the minimal loss. The candidate touch event achieving the lowest loss (i.e., a minimal error) between may correspond to the estimated touch location. As another example, the one or more processors 150 may implement a known gradient descent method to obtain a result (X, Y coordinate) which achieves the minimal loss. The gradient descent search method may utilize random candidate touch events (i.e., random candidate X, Y coordinates) to obtain a result which obtains the minimal loss rather than a direct search report in which all of the candidate touch events are tested. The gradient descent search method may use a smaller search space and thus be less computationally expensive than the direct search approach, saving energy and time. The disclosure is not limited to a direct search method or gradient descent search method, and other methods for minimizing a loss function may also be implemented.

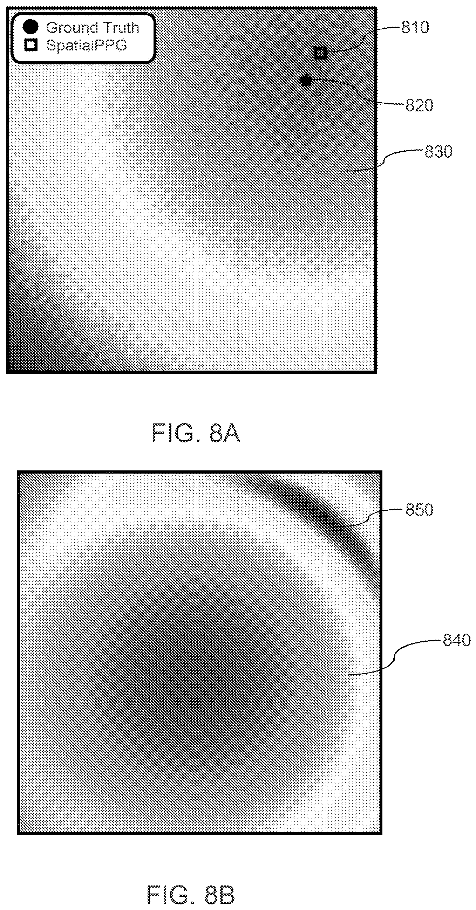

FIGS. 8A and 8B illustrate an example estimate of the location of a force applied to the display based on a loss function result, according to one or more example embodiments of the disclosure. For example, in FIG. 8A, an estimated location of a force applied to the display 182 (upper right corner) is denoted by reference numeral 810. The location of the force has been calculated or determined by the one or more processors 150 using values output by detectors configured as in the example of FIG. 4C. The ground truth (actual) location of the force is denoted by reference numeral 820. The Gaussian press response 830 is also shown in the background to provide additional context. For example, FIG. 8B illustrates a corresponding loss function result 840 where an outer band 850 denotes the most likely location of a minimizing result, in which the estimated location 810 also corresponds.

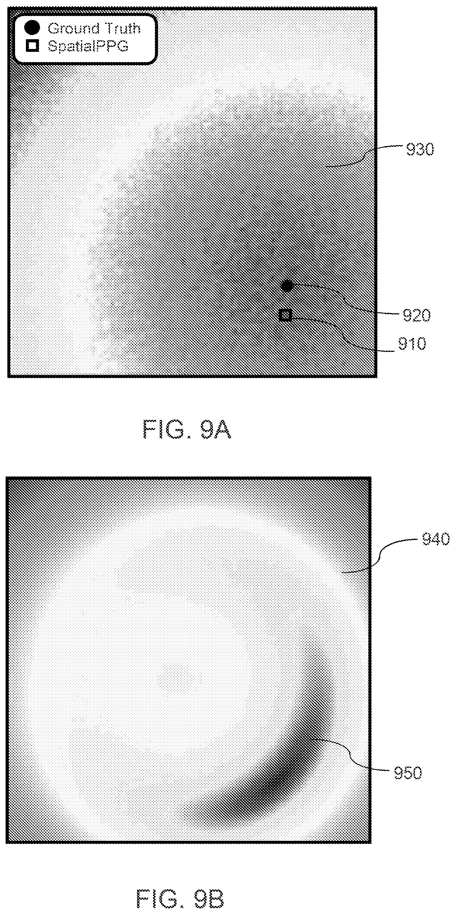

FIGS. 9A and 9B illustrate another example estimate of the location of a force applied to the display based on a loss function result, according to one or more example embodiments of the disclosure. For example, in FIG. 9A, an estimated or determined location of a force applied to the display 182 (lower right corner) is denoted by reference numeral 910. The location of the force has been calculated or determined by the one or more processors 150 using values output by detectors configured as in the example of FIG. 4C. The ground truth (actual) location of the force is denoted by reference numeral 920. The Gaussian press response 930 is also shown in the background to provide additional context. For example, FIG. 9B illustrates a corresponding loss function result 940 where an outer band 950 denotes the most likely location of a minimizing result, in which the estimated location 910 also corresponds.

In some circumstances, noise may affect the output of the PPG sensors 172. For example, a noise level experienced by the PPG sensors 172 may increase when the wearable computing device 100 is not securely held to a body part of the user. In another example, a noise level experienced by the PPG sensors 172 may increase due to a low battery condition where the amount of light output by the light source (e.g., LED 172d) is decreased (e.g., a low power mode). When noise is experienced by the PPG sensors 172, the optical readings may be affected by the noise, and the accuracy of the PPG sensors 172 may be reduced.

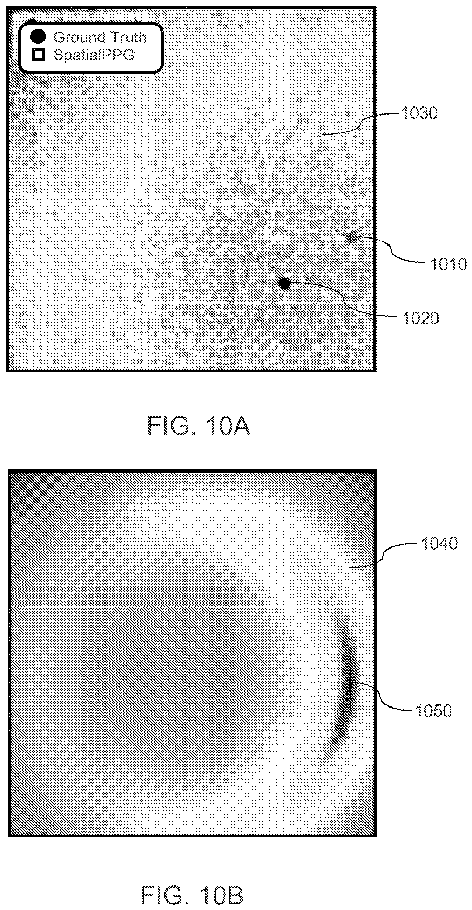

FIGS. 10A and 10B illustrate an example estimate of the location of a force applied to the display based on a loss function result in a noisy environment, according to one or more example embodiments of the disclosure. For example, in FIG. 10A, an estimated location of a force applied to the display 182 (lower right corner) is denoted by reference numeral 1010, and is located further away from the ground truth value 1020 compared to the example of FIG. 9A. The location of the force has been calculated or determined by the one or more processors 150 using values output by detectors configured as in the example of FIG. 4C. The Gaussian press response 1030 is also shown in the background to provide additional context. For example, FIG. 10B illustrates a corresponding loss function result 1040 where an outer band 1050 denotes the most likely location of a minimizing result, in which the estimated location 1010 also corresponds.

In one or more example embodiments, in response to a noise level of the PPG sensors 172 increasing beyond a threshold, the one or more processors 150 may be configured to control the display 182 so that a number of the plurality of selectable functions displayed on the display 182 may be reduced. For example, functions F1-F4 displayed on the display 182 in FIG. 4A may be reduced to display only functions F1 (on the upper half of the display 182) and F2 (on the lower half of the display 182). Thus, a number of the plurality of selectable functions displayed on the display 182 may be reduced when the noise level of the sensors exceeds a threshold (e.g., reducing icons displayed on the display 182 from ten icons to four icons). Stopping the displaying of one or more of the plurality of selectable functions (i.e., reducing the number of the plurality of selectable functions displayed on the display 182) may enlarge a space on the display 182 for each of the remaining selectable functions to be displayed. Thus, a likelihood that the one or more processors 150 may accurately execute a function that the user intends to select may be increased, even though an accuracy of the detection or determination of the location of the force applied to the display 182 may be reduced due to the noise encountered or experienced by the PPG sensors 172. In addition, by changing (reducing) the number of selectable functions displayed on the display 182 to increase the accuracy of the determination of the location), the noisy condition encountered by the PPG sensors 172 may be masked and go unnoticed by the user, thereby improving the user experience.

In one or more examples, the wearable computing device 100 may include an accelerometer 174 and/or a gyroscope 176 to detect or sense motion data of the wearable computing device 100. As described herein, the wearable computing device 100 may include PPG sensors 172 which are configured to monitor a heart rate of the user when the user wears the wearable computing device 100.

Motion of a user's hand affects arterial pressure and volumetric structure (muscle/tendon), creating a signature dip in reflection/absorption reflected in the PPG signal. The PPG sensors 172 may capture the arterial pressure profiles spatially over a two-dimensional area (e.g., in x and y directions) on the lower side 110b of the housing 110 of the wearable computing device 100. The one or more processors 150 may be configured to identify a gesture of the user based on the one or more optical readings output by the PPG sensors 172, and the one or more processors 150 may be configured to execute a function of the computer wearable device 100 (or another device) based on the gesture of the user identified by the one or more processors 150. The one or more processors 150 may be configured to identify the gesture of the user based on the one or more optical readings output by the PPG sensors 172 together with one or more outputs of the accelerometer 174 and/or the gyroscope 176. The outputs of the accelerometer 174 and/or the gyroscope 176 may additionally or alternatively be used to confirm the validity of the outputs of the PPG sensors 172 that a gesture is being performed by the user.

In one or more examples, the gesture of the user may include hand gestures including clinching of a fist, a pinching gesture with the fingers, squeezing of a hand, and the like. The gestures may be mapped to respective functions of the wearable computing device (e.g., to make a phone call), or a function of another device (e.g., to operate a home appliance) which may be controllable via the wearable computing device 100 by the identification of the gesture. In one or more examples, the one or more processors 150 may identify a gesture based on the one or more optical readings output by the PPG sensors 172. For example, each gesture may correspond to a respective optical signal output by the PPG sensors 172 so that a gesture may be correctly identified. In an example, optical signals output by the PPG sensors 172 may be analyzed to identify the gesture (e.g., via machine learning algorithms to classify the output optical readings, by comparing the optical signals with known optical signals corresponding to respective gestures, etc.). To more accurately analyze the optical signals, portions of the optical readings which are attributed to a heart rate of the user may be filtered out. For example, a high pass heart rate filtering operation may be performed with respect to the optical readings to notch out the heart rate (arterial pulse), so that the majority of the optical readings which are used to identify the gesture corresponds to or is attributed to, the movement of the user's hand.

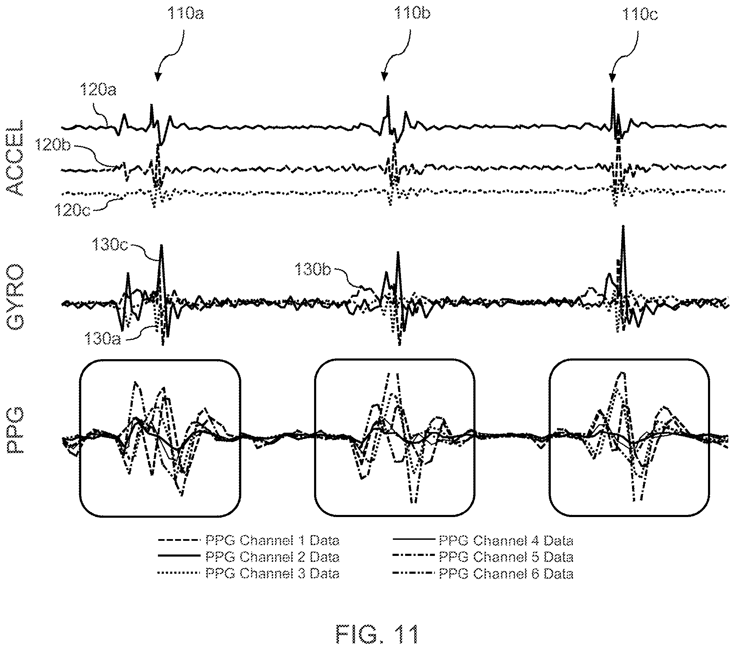

FIG. 11 illustrates example response signals of various sensors of the wearable computing device to detect a gesture of a user, according to one or more example embodiments of the disclosure. In FIG. 11, when a user makes a pinch gesture at times corresponding to reference characters 1110a, 1110b, and 1110c, a distinctive or significant change in the output signals of the accelerometer 174, gyroscope 176, and PPG sensors 172 occurs. The PPG signals shown in FIG. 11 are obtained after high-pass heart rate filtering which results in a distinctive wavelet-like pattern in the PPG signal.

For example, in FIG. 11 motion information / data is collected via the accelerometer along three axes (Y-axis 1120a, X-axis 1120b, and Z-axis 1120c). For example, in FIG. 11 motion information / data is collected via the gyroscope along three axes (Y-axis 1130a, X-axis 1130b, and Z-axis 1130c). For example, in FIG. 11 the PPG optical readings includes information / data collected from six channels: PPG channel 1 data 1140a, PPG channel 2 data 1140b, PPG channel 3 data 1140c, channel 4 data 1140d, PPG channel 5 data 1140e, and PPG channel 6 data 1140f. The signals output by the accelerometer 174 and/or gyroscope 176 may be analyzed in conjunction with the signals output by the PPG sensors 172 to determine the gesture of the user's hand (e.g., the pinch gesture). However, in some examples only the signals output by the PPG sensors 172 may be used to determine the gesture of the user's hand. Based on the determination of the gesture, a corresponding function of the wearable computing device 100 and/or of an external device 190 may be performed.

For example, a plurality of PPG optical readings may be obtained through a multiplexing operation. For example, a plurality of LEDs may also be used to obtain a plurality of optical readings from different combinations of the detectors and the LEDs, to thereby obtain spatial backscatter diversity. For example, in a configuration in which three LEDs and three detectors are included in the PPG sensor 172, in a multiplexing operation in which a first LED is turned on (illuminated), each of the three detectors may receive reflected light and output a respective signal. When a second LED is next turned on, each of the three detectors may receive reflected light and output a respective second signal, and when a third LED is lastly turned on, each of the three detectors may receive reflected light and output a respective third signal. In such a configuration a total of nine PPG optical readings may be obtained to provide a spatial perfusion index profile change for accurate classification of hand states. Also, as discussed above, the signals output by the accelerometer 174 and/or gyroscope 176 may be analyzed in conjunction with the signals output by the PPG sensors 172 to determine the gesture of the user's hand. However, in some examples only the signals output by the PPG sensors 172 may be used to determine the gesture of the user's hand.

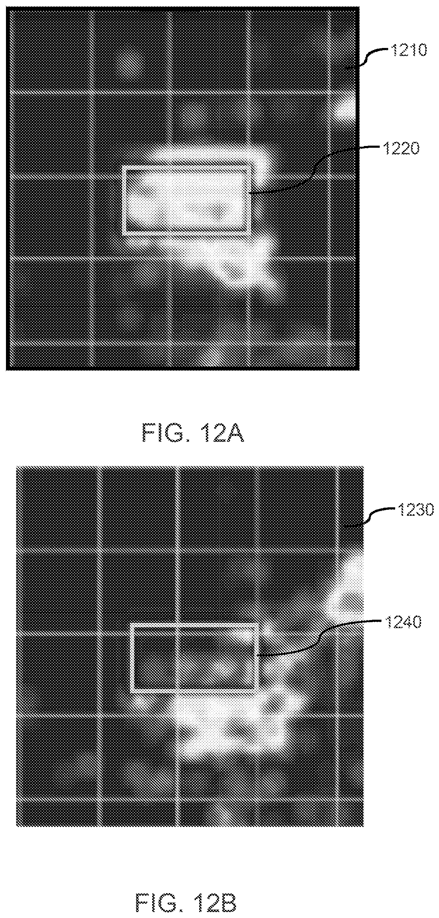

FIGS. 12A and 12B depict example one-handed gestures of a user and corresponding heatmaps reconstructed from output optical readings from the PPG sensors, according to one or more example embodiments of the disclosure. In FIG. 12A, when a user makes a first gesture (e.g. by tilting a hand upward) a first heat map 1210 is generated, and in FIG. 12B when a user makes a second gesture (e.g. by tilting a hand downward) a second heat map 1230 is generated. As can be seen by comparing FIG. 12A with a FIG. 12B, a heat signature corresponding to the portion 1220 in FIG. 12A is very different from a heat signature corresponding to the portion 1240 in FIG. 12B. Each gesture may have a unique or identifiable heat map that can be correlated with the output optical readings from the PPG sensors 172, so that the wearable computing device 100 can identify or determine a gesture based on the output optical readings from the PPG sensors 172. The determination of the gesture may be performed using the probability density of a standard Gaussian function which may be used to determine the gesture using measurements obtained from the detectors 172a-172c, which are sparsely provided and distributed, similar to the method described above for determining a location of a touch on a user interface 180 of the wearable computing device 100. Based on the determination of the gesture, a corresponding function of the wearable computing device 100 and/or of an external device 190 may be performed.

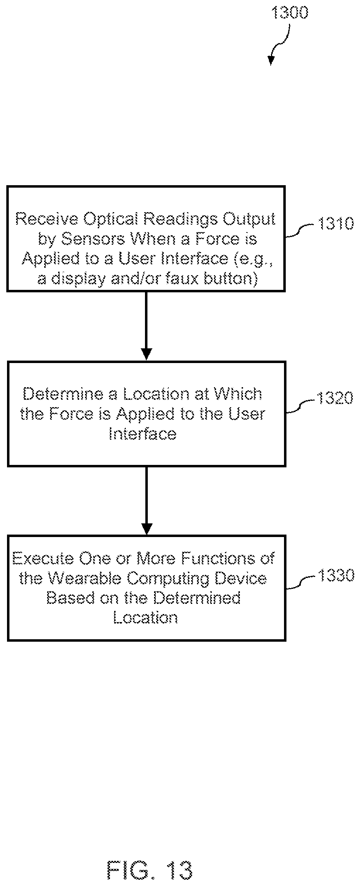

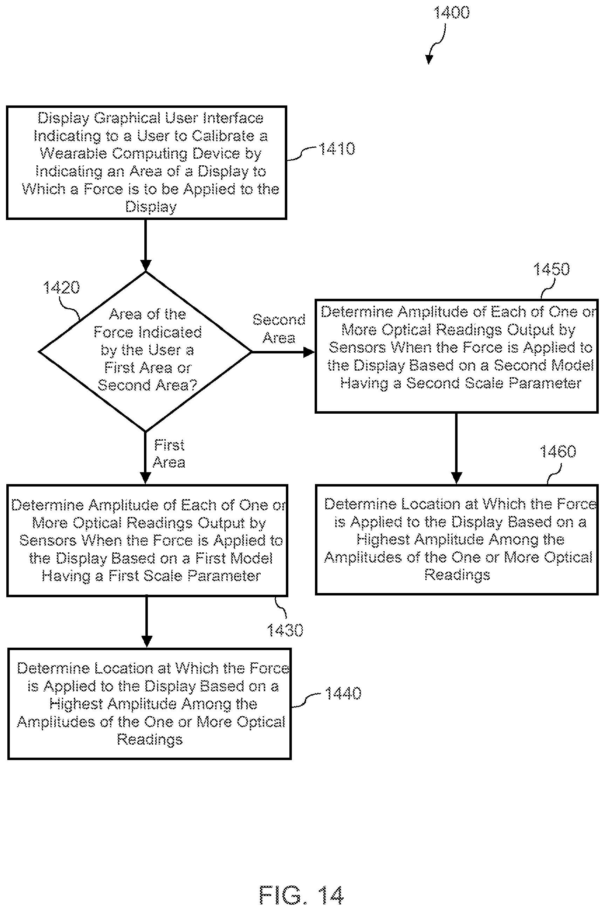

Examples of the disclosure are also directed to computer implemented methods of a wearable computing device. FIGS. 13 and 14 each illustrate a flow diagram of an example, non-limiting computer-implemented method according to one or more example embodiments of the disclosure.

Referring to FIG. 13, in an example computer implemented method 1300 at operation 1310 one or more processors 150 may receive optical readings (signals) output by PPG sensors 172 when a force is applied to a user interface 180 (e.g., including a display and/or one or more faux buttons 184a, 184b) disposed on an upper side 110a of the housing 110. The PPG sensors 172 are disposed on a lower side 110b of the housing 110 which is disposed opposite to the upper side 110a of the housing 110 (e.g., as shown in FIG. 2 of the drawings). The lower side 110b of the housing 110 may be configured to be in contact with a body part of a user when the wearable computing device 100 is worn by the user. At operation 1320 the method may further include determining, by one or more processors 150 of the wearable computing device 100, a location at which the force is applied to the user interface 180 based on the one or more received optical readings. At operation 1330 the method may further include the one or more processors 150 executing one or more functions of the wearable computing device 100 based on the determined location at which the force is applied to the user interface 180. Example functions of the wearable computing device 100 may include checking health information about the user such as a blood pressure, making and/or receiving a phone call, sending and/or receiving a text message, obtaining a current time, setting a timer, a stopwatch function, controlling an external device 190 such as a home appliance, an electronic device such as a television, and the like.

Referring to FIG. 14, in an example computer implemented method 1400 at operation 1410 a graphical user interface is displayed on the display 182 indicating to a user to calibrate the wearable computing device 100 by indicating an area of the display 182 to which a force is to be applied to the display 182. For example, the calibration instructions may ask the user whether the user prefers to use an index finger (second area) to provide inputs or a thumb (first area). For example, in addition or alternatively, the calibration instructions may ask the user whether the user is male or female. For example, in addition or alternatively, the calibration instructions may ask the user whether the user has hands and/or fingers which are larger than a threshold value which may imply that a first area should be set (larger than the threshold value) or a second area should be set (equal to or less than the threshold value). For example, if a user has a ring size greater than ten, a first area setting may be assumed. For example, if a user has a finger length greater than 7.5 inches (measured from the tip of the longest finger to the crease under the palm), a first area setting may be assumed. At operation 1420 the user may provide an input (e.g., via one or more buttons 184c located on a periphery of the wearable computing device 100 as shown in FIG. 1), indicating an area of the force as a first area or a second area.

When the area of the force indicated by the user corresponds to the first area, the method may include operation 1430, which includes determining an amplitude of each of the one or more optical readings based on a first model having a first scale parameter k1 which reflects a first variance of the force to be applied to the display 182. When the area of the force indicated by the user corresponds to a second area, the method may include operation 1450, which is determining the amplitude of each of the one or more optical readings based on a second model having a second scale parameter k2 which reflects a second variance of the force to be applied to the display 182. For example, the first area may be greater than the second area, and the first variance may be greater than the second variance. At each of operations 1440 and 1460, the method may include the one or more processors 150 determining the location at which the force is applied to the display 182 based on a highest amplitude among the amplitudes of the one or more optical readings. The method may also include determining the location at which the force is applied to the display 182 based on an optical reading of a PPG sensor 172 among the PPG sensors 172 having a highest value, among the one or more optical readings received by the one or more processors 150.

Other operations of the calibration procedure may include asking the user to apply a force to the display 182 at various known (predetermined) points of the display 182, and saving the signals which are output in response to memory 160 as data 162 which may be used as a lookup table for comparing future output signals obtained from PPG sensors 172 and/or for guiding a decision in determining the location of an application of force according to the loss function as described herein.

Aspects of the above-described example embodiments may be recorded in non-transitory computer-readable media including program instructions to implement various operations embodied by a computer. The media may also include, alone or in combination with the program instructions, data files, data structures, and the like. Examples of non-transitory computer-readable media include magnetic media such as hard disks, floppy disks, and magnetic tape; optical media such as CD ROM disks, Blue-Ray disks, and DVDs; magneto-optical media such as optical discs; and other hardware devices that are specially configured to store and perform program instructions, such as semiconductor memory, read-only memory (ROM), random access memory (RAM), flash memory, USB memory, and the like. Examples of program instructions include both machine code, such as produced by a compiler, and files containing higher level code that may be executed by the computer using an interpreter. The program instructions may be executed by one or more processors. The described hardware devices may be configured to act as one or more software modules in order to perform the operations of the above-described embodiments, or vice versa. In addition, a non-transitory computer-readable storage medium may be distributed among computer systems connected through a network and computer-readable codes or program instructions may be stored and executed in a decentralized manner. In addition, the non-transitory computer-readable storage media may also be embodied in at least one application specific integrated circuit (ASIC) or Field Programmable Gate Array (FPGA).

Each block of the flowchart illustrations may represent a unit, module, segment, or portion of code, which comprises one or more executable instructions for implementing the specified logical function(s). It should also be noted that in some alternative implementations, the functions noted in the blocks may occur out of order. For example, two blocks shown in succession may in fact be executed substantially concurrently (simultaneously) or the blocks may sometimes be executed in the reverse order, depending upon the functionality involved.

While the disclosure has been described with respect to various example embodiments, each example is provided by way of explanation, not limitation of the disclosure.

## Claims

1. A wearable computing device, comprising:
a housing (110) including an upper side (110a) and a lower side (110b), wherein the lower side of the housing is opposite to the upper side of the housing and is configured to be in contact with a body part (102) of a user when the wearable computing (100) device is worn by the user;
a user interface (180) disposed on the upper side of the housing;
sensors (170), disposed on the lower side of the housing, configured to output one or more optical readings (S1, S2, S3) when a force is applied to the user interface, wherein the sensors include photoplethysmography, PPG, sensors (172) configured to monitor a heart rate of the user when the wearable computing device is worn by the user, and the PPG sensors include one or more light-emitting diodes and a plurality of detectors (172a, 172b, 172c); and
one or more processors (150) configured to determine a location at which the force is applied to the user interface based on the one or more optical readings output by the PPG sensors.

2. The wearable computing device according to claim 1, wherein
the sensors include at least three PPG sensors, and
at least one PPG sensor of the at least three PPG sensors is spaced apart from another PPG sensor of the at least three PPG sensors in a first direction and a second direction.

3. The wearable computing device according to claim 1, wherein
the one or more processors are configured to execute one or more functions of the wearable computing device based on the location at which the force is applied to the user interface as determined by the one or more processors.

4. The wearable computing device according to claim 1, wherein
the one or more processors are configured to determine the location at which the force is applied to the user interface based on whether an area of the user interface to which the force is applied corresponds to a first area or a second area, the first area being different from the second area.

5. The wearable computing device according to claim 4, wherein:
when the area of the user interface to which the force is applied corresponds to the first area, the one or more processors are configured to determine an amplitude of each of the one or more optical readings based on a first model having a first scale parameter (k1) which reflects a first variance of the force applied to the user interface, and
when the area of the user interface to which the force is applied corresponds to the second area, the one or more processors are configured to determine the amplitude of each of the one or more optical readings based on a second model having a second scale parameter (k2) which reflects a second variance of the force applied to the user interface, the second variance being different from the first variance.

6. The wearable computing device according to claim 5, wherein
determining the location at which the force is applied to the user interface comprises using a value of each of the one or more optical readings output by the sensors and known positions of the sensors to estimate the location at which the force is applied by applying a loss function to minimize a difference between an actual location at which the force is applied and an estimated location at which the force is applied, wherein the loss function includes a probability density function determining a probability of an optical reading output by an optical sensor being corrupted by measurement noises.

7. The wearable computing device according to claim 6, wherein
the one or more processors are configured to apply the loss function by performing a two-dimensional grid search method or a gradient descent search method.

8. The wearable computing device according to claim 1, wherein
a)
the user interface includes a non-touch sensitive display (182) configured to display a plurality of elements corresponding to a plurality of selectable functions (F3, F4) of the wearable computing device,
in response to a noise level of the sensors increasing beyond a threshold, the one or more processors are configured to control the non-touch sensitive display to stop displaying of one or more of the plurality of elements, and
in response to determining the location at which the force is applied to the non-touch sensitive display corresponds to a remaining first element of the plurality of elements displayed on the non-touch sensitive display, the one or more processors are further configured to execute a first selectable function of the plurality of selectable functions which corresponds to the remaining first element, based on the determined location; and/or
b)
the user interface includes a plurality of faux buttons (184a, 184b) which respectively correspond to a plurality of selectable functions (F3, F4) of the wearable computing device, and
in response to determining the location at which the force is applied to a first faux button of the plurality of faux buttons of the user interface, the one or more processors are configured to execute a first selectable function of the plurality of selectable functions which corresponds to the first faux button, based on the determined location.

9. The wearable computing device according to claim 1, wherein
the one or more processors are configured to identify a gesture of the user based on the one or more optical readings output by the PPG sensors, and
the one or more processors are configured to execute a function of the computer wearable device based on the gesture of the user identified by the one or more processors.

10. The wearable computing device according to claim 9, further comprising:
at least one of an accelerometer (174) or a gyroscope (176),
wherein
the one or more processors are configured to identify the gesture of the user based on the one or more optical readings output by the PPG sensors and one or more outputs of the at least one of the accelerometer or the gyroscope.

11. A computer-implemented method, comprising:
receiving one or more optical readings (S1, S2, S3) output by sensors (170) disposed on a lower side (110b) of a housing (110) of a wearable computing device (100), when a force is applied to a user interface (180) disposed on an upper side (110a) of the housing, wherein the lower side of the housing is opposite to the upper side of the housing and is configured to be in contact with a body part (102) of a user when the wearable computing device is worn by the user, and wherein the sensors include photoplethysmography, PPG, sensors (172) configured to monitor a heart rate of the user when the wearable computing device is worn by the user, and the PPG sensors include one or more light-emitting diodes and a plurality of detectors (172a, 172b, 172c); and
determining, by one or more processors (150) of the wearable computing device, a location at which the force is applied to the user interface based on the one or more received optical readings output by the PPG sensors.

12. The computer-implemented method of claim 11, further comprising:
executing one or more functions (F3, F4) of the wearable computing device based on the location at which the force is applied to the user interface as determined by the one or more processors.

13. The computer-implemented method of claim 11, further comprising:
displaying on a display (182) of the user interface instructions indicating to a user to calibrate the wearable computing device by indicating an area of the display to which the force is to be applied to the display.

14. The computer-implemented method of claim 13, further comprising:
when the area of the force indicated by the user corresponds to a first area, determining an amplitude of each of the one or more optical readings based on a first model having a first scale parameter (k1) which reflects a first variance of the force to be applied to the display, and
when the area of the force indicated by the user corresponds to a second area, determining the amplitude of each of the one or more optical readings based on a second model having a second scale parameter (k2) which reflects a second variance of the force to be applied to the display, the first area being greater than the second area, and the first variance being greater than the second variance.

15. The computer-implemented method of claim 11, determining the location at which the force is applied to the user interface comprises using a value of each of the one or more optical readings output by the sensors and known positions of the sensors to estimate the location at which the force is applied by applying a loss function to minimize a difference between an actual location at which the force is applied and an estimated location at which the force is applied, wherein the loss function includes a probability density function determining a probability of an optical reading output by an optical sensor being corrupted by measurement noises.

## Patentansprüche

1. Tragbare Rechenvorrichtung, umfassend:
ein Gehäuse (110), beinhaltend eine Oberseite (110a) und eine Unterseite (110b), wobei die Unterseite des Gehäuses der Oberseite des Gehäuses gegenüberliegend und konfiguriert ist, um mit einem Körperteil (102) eines Benutzers in Kontakt zu sein, wenn die tragbare Rechenvorrichtung (100) von dem Benutzer getragen wird;
eine Benutzerschnittstelle (180), die auf der Oberseite des Gehäuses angeordnet ist;
Sensoren (170), die auf der Unterseite des Gehäuses angeordnet sind, konfiguriert, einen oder mehrere optische Messwerte (S1, S2, S3) auszugeben, wenn eine Kraft auf die Benutzerschnittstelle angewendet wird, wobei die Sensoren Photoplethysmographie-, PPG-, Sensoren (172) beinhalten, die konfiguriert sind, um eine Herzfrequenz des Benutzers zu überwachen, wenn die tragbare Rechenvorrichtung von dem Benutzer getragen wird, und die PPG-Sensoren eine oder mehrere Leuchtdioden und eine Vielzahl von Detektoren (172a, 172b, 172c) beinhalten; und
einen oder mehrere Prozessoren (150), die konfiguriert sind, um eine Stelle zu bestimmen, an der die Kraft auf die Benutzerschnittstelle angewendet wird, basierend auf dem einen oder den mehreren optischen Messwerten, die von den PPG-Sensoren ausgegeben werden.

2. Tragbare Rechenvorrichtung nach Anspruch 1, wobei
die Sensoren mindestens drei PPG-Sensoren beinhalten, und
mindestens ein PPG-Sensor der mindestens drei PPG-Sensoren von einem anderen PPG-Sensor der mindestens drei PPG-Sensoren in einer ersten Richtung und einer zweiten Richtung beabstandet ist.

3. Tragbare Rechenvorrichtung nach Anspruch 1, wobei
der eine oder die mehreren Prozessoren konfiguriert sind, um eine oder mehrere Funktionen der tragbaren Rechenvorrichtung basierend auf der Stelle, an der die Kraft auf die Benutzerschnittstelle angewendet wird, wie von dem einen oder den mehreren Prozessoren bestimmt, auszuführen.

4. Tragbare Rechenvorrichtung nach Anspruch 1, wobei
der eine oder die mehreren Prozessoren konfiguriert sind, um die Stelle zu bestimmen, an der die Kraft auf die Benutzerschnittstelle angewendet wird, basierend darauf, ob ein Bereich der Benutzerschnittstelle, auf den die Kraft angewendet wird, einem ersten Bereich oder einem zweiten Bereich entspricht, wobei der erste Bereich von dem zweiten Bereich verschieden ist.

5. Tragbare Rechenvorrichtung nach Anspruch 4, wobei:
wenn der Bereich der Benutzerschnittstelle, auf den die Kraft angewendet wird, dem ersten Bereich entspricht, der eine oder die mehreren Prozessoren konfiguriert sind, um eine Amplitude jedes des einen oder der mehreren optischen Messwerte basierend auf einem ersten Modell zu bestimmen, das einen ersten Skalierungsparameter (k1) aufweist, der eine erste Varianz der auf die Benutzerschnittstelle angewendeten Kraft widerspiegelt, und
wenn der Bereich der Benutzerschnittstelle, auf den die Kraft angewendet wird, dem zweiten Bereich entspricht, der eine oder die mehreren Prozessoren konfiguriert sind, um die Amplitude jedes des einen oder der mehreren optischen Messwerte basierend auf einem zweiten Modell zu bestimmen, das einen zweiten Skalierungsparameter (k2) aufweist, der eine zweite Varianz der auf die Benutzerschnittstelle angewendeten Kraft widerspiegelt, wobei die zweite Varianz von der ersten Varianz verschieden ist.

6. Tragbare Rechenvorrichtung nach Anspruch 5, wobei
Bestimmen der Stelle, an der die Kraft auf die Benutzerschnittstelle angewendet wird, umfasst, einen Wert jedes des einen oder der mehreren optischen Messwerte, die von den Sensoren ausgegeben werden, und bekannte Positionen der Sensoren zu verwenden, um die Stelle zu schätzen, an der die Kraft angewendet wird, durch Anwenden einer Verlustfunktion zum Minimieren einer Differenz zwischen einer tatsächlichen Stelle, an der die Kraft angewendet wird, und einer geschätzten Stelle, an der die Kraft angewendet wird, wobei die Verlustfunktion eine Wahrscheinlichkeitsdichtefunktion beinhaltet, die eine Wahrscheinlichkeit bestimmt, dass ein optischer Messwert, der von einem optischen Sensor ausgegeben wird, durch Messrauschen verfälscht ist.

7. Tragbare Rechenvorrichtung nach Anspruch 6, wobei
der eine oder die mehreren Prozessoren konfiguriert sind, um die Verlustfunktion durch Durchführen eines zweidimensionalen Gittersuchverfahrens oder eines Gradientenabstiegssuchverfahrens anzuwenden.

8. Tragbare Rechenvorrichtung nach Anspruch 1, wobei
a)
die Benutzerschnittstelle ein nicht berührungsempfindliches Display (182) beinhaltet, das konfiguriert ist, um eine Vielzahl von Elementen anzuzeigen, die einer Vielzahl von auswählbaren Funktionen (F3, F4) der tragbaren Rechenvorrichtung entsprechen, als Reaktion darauf, dass ein Rauschpegel der Sensoren über einen Schwellenwert hinaus ansteigt, der eine oder die mehreren Prozessoren konfiguriert sind, um das nicht berührungsempfindliche Display zu steuern, Anzeigen eines oder mehrerer der Vielzahl von Elementen zu stoppen, und
als Reaktion auf Bestimmen, dass die Stelle, an der die Kraft auf das nicht berührungsempfindliche Display angewendet wird, einem verbleibenden ersten Element der Vielzahl von Elementen entspricht, die auf dem nicht berührungsempfindlichen Display angezeigt werden, der eine oder die mehreren Prozessoren ferner konfiguriert sind, um eine erste auswählbare Funktion der Vielzahl von auswählbaren Funktionen, die dem verbleibenden ersten Element entspricht, basierend auf der bestimmten Stelle auszuführen; und/oder
b)
die Benutzerschnittstelle eine Vielzahl von Scheintasten (184a, 184b) beinhaltet, die jeweils einer Vielzahl von auswählbaren Funktionen (F3, F4) der tragbaren Rechenvorrichtung entsprechen, und
als Reaktion auf Bestimmen der Stelle, an der die Kraft auf eine erste Scheintaste der Vielzahl von Scheintasten der Benutzerschnittstelle angewendet wird, der eine oder die mehreren Prozessoren konfiguriert sind, um eine erste auswählbare Funktion der Vielzahl von auswählbaren Funktionen, die der ersten Scheintaste entspricht, basierend auf der bestimmten Stelle auszuführen.

9. Tragbare Rechenvorrichtung nach Anspruch 1, wobei
der eine oder die mehreren Prozessoren konfiguriert sind, um eine Geste des Benutzers basierend auf dem einen oder den mehreren optischen Messwerten, die von den PPG-Sensoren ausgegeben werden, zu identifizieren, und
der eine oder die mehreren Prozessoren konfiguriert sind, um eine Funktion der tragbaren Computer-Vorrichtung basierend auf der Geste des Benutzers, die von dem einen oder den mehreren Prozessoren identifiziert wird, auszuführen.

10. Tragbare Rechenvorrichtung nach Anspruch 9, ferner umfassend:
mindestens eines von einem Beschleunigungsmesser (174) oder einem Gyroskop (176),
wobei
der eine oder die mehreren Prozessoren konfiguriert sind, um die Geste des Benutzers basierend auf dem einen oder den mehreren optischen Messwerten, die von den PPG-Sensoren ausgegeben werden, und einer oder mehreren Ausgaben des mindestens einen von dem Beschleunigungsmesser oder dem Gyroskop zu identifizieren.

11. Computerimplementiertes Verfahren, umfassend:
Empfangen eines oder mehrerer optischer Messwerte (S1, S2, S3), die von Sensoren (170) ausgegeben werden, die auf einer Unterseite (110b) eines Gehäuses (110) einer tragbaren Rechenvorrichtung (100) angeordnet sind, wenn eine Kraft auf eine Benutzerschnittstelle (180) angewendet wird, die auf einer Oberseite (110a) des Gehäuses angeordnet ist, wobei die Unterseite des Gehäuses der Oberseite des Gehäuses gegenüberliegend und konfiguriert ist, um mit einem Körperteil (102) eines Benutzers in Kontakt zu sein, wenn die tragbare Rechenvorrichtung von dem Benutzer getragen wird, und wobei die Sensoren Photoplethysmographie-, PPG-, Sensoren (172) beinhalten, die konfiguriert sind, um eine Herzfrequenz des Benutzers zu überwachen, wenn die tragbare Rechenvorrichtung von dem Benutzer getragen wird, und die PPG-Sensoren eine oder mehrere Leuchtdioden und eine Vielzahl von Detektoren (172a, 172b, 172c) beinhalten; und
Bestimmen, durch einen oder mehrere Prozessoren (150) der tragbaren Rechenvorrichtung, einer Stelle, an der die Kraft auf die Benutzerschnittstelle angewendet wird, basierend auf den einen oder mehreren empfangenen optischen Messwerten, die durch die PPG-Sensoren ausgegeben werden.

12. Computerimplementiertes Verfahren nach Anspruch 11, ferner umfassend:
Ausführen einer oder mehrerer Funktionen (F3, F4) der tragbaren Rechenvorrichtung basierend auf der Stelle, an der die Kraft auf die Benutzerschnittstelle angewendet wird, wie durch den einen oder die mehreren Prozessoren bestimmt.

13. Computerimplementiertes Verfahren nach Anspruch 11, ferner umfassend:
Anzeigen auf einem Display (182) der Benutzerschnittstelle von Anweisungen, die einem Benutzer anzeigen, die tragbare Rechenvorrichtung zu kalibrieren, indem ein Bereich des Displays angegeben wird, auf den die Kraft auf das Display anzuwenden ist.

14. Computerimplementiertes Verfahren nach Anspruch 13, ferner umfassend:
wenn der Bereich der Kraft, der durch den Benutzer angegeben wird, einem ersten Bereich entspricht, Bestimmen einer Amplitude jedes der einen oder mehreren optischen Messwerte basierend auf einem ersten Modell, das einen ersten Skalierungsparameter (k1) aufweist, der eine erste Varianz der auf das Display anzuwendenden Kraft widerspiegelt, und
wenn der Bereich der Kraft, der durch den Benutzer angegeben wird, einem zweiten Bereich entspricht, Bestimmen der Amplitude jedes der einen oder mehreren optischen Messwerte basierend auf einem zweiten Modell, das einen zweiten Skalierungsparameter (k2) aufweist, der eine zweite Varianz der auf das Display anzuwendenden Kraft widerspiegelt, wobei der erste Bereich größer als der zweite Bereich ist und die erste Varianz größer als die zweite Varianz ist.

15. Computerimplementiertes Verfahren nach Anspruch 11, wobei Bestimmen der Stelle, an der die Kraft auf die Benutzerschnittstelle angewendet wird, Verwenden eines Werts jedes der einen oder mehreren optischen Messwerte, die durch die Sensoren ausgegeben werden, und bekannter Positionen der Sensoren umfasst, um die Stelle, an der die Kraft angewendet wird, zu schätzen, indem eine Verlustfunktion angewendet wird, um eine Differenz zwischen einer tatsächlichen Stelle, an der die Kraft angewendet wird, und einer geschätzten Stelle, an der die Kraft angewendet wird, zu minimieren, wobei die Verlustfunktion eine Wahrscheinlichkeitsdichtefunktion beinhaltet, die eine Wahrscheinlichkeit bestimmt, dass ein optischer Messwert, der durch einen optischen Sensor ausgegeben wird, durch Messrauschen verfälscht ist.

## Revendications

1. Dispositif informatique portable, comprenant :
un boîtier (110) comportant un côté supérieur (110a) et un côté inférieur (110b), dans lequel le côté inférieur du boîtier est opposé au côté supérieur du boîtier et est configuré pour être en contact avec une partie de corps (102) d'un utilisateur lorsque le dispositif informatique portable (100) est porté par l'utilisateur ;
une interface utilisateur (180) disposée sur le côté supérieur du boîtier ;
des capteurs (170), disposés sur le côté inférieur du boîtier, configurés pour produire une ou plusieurs lectures optiques (S1, S2, S3) lorsqu'une force est appliquée sur l'interface utilisateur, dans lequel les capteurs comportent des capteurs de photopléthysmographie, PPG, (172) configurés pour surveiller une fréquence cardiaque de l'utilisateur lorsque le dispositif informatique portable est porté par l'utilisateur, et les capteurs PPG comportent une ou plusieurs diodes électroluminescentes et une pluralité de détecteurs (172a, 172b, 172c) ; et
un ou plusieurs processeurs (150) configurés pour déterminer une localisation où la force est appliquée à l'interface utilisateur sur la base des une ou plusieurs lectures optiques produites par les capteurs PPG.

2. Dispositif informatique portable selon la revendication 1, dans lequel
les capteurs comportent au moins trois capteurs PPG, et
au moins un capteur PPG parmi les au moins trois capteurs PPG est espacé d'un autre capteur PPG parmi les au moins trois capteurs PPG dans une première direction et une seconde direction.

3. Dispositif informatique portable selon la revendication 1, dans lequel
les un ou plusieurs processeurs sont configurés pour exécuter une ou plusieurs fonctions du dispositif informatique portable sur la base de la localisation où la force est appliquée à l'interface utilisateur tel que déterminé par les un ou plusieurs processeurs.

4. Dispositif informatique portable selon la revendication 1, dans lequel
les un ou plusieurs processeurs sont configurés pour déterminer la localisation où la force est appliquée à l'interface utilisateur sur la base du fait qu'une zone de l'interface utilisateur à laquelle la force est appliquée correspond à une première zone ou à une seconde zone, la première zone étant différente de la seconde zone.

5. Dispositif informatique portable selon la revendication 4, dans lequel :
lorsque la zone de l'interface utilisateur à laquelle la force est appliquée correspond à la première zone, les un ou plusieurs processeurs sont configurés pour déterminer une amplitude de chacune des une ou plusieurs lectures optiques sur la base d'un premier modèle ayant un premier paramètre d'échelle (k1) qui reflète une première variance de la force appliquée à l'interface utilisateur, et
lorsque la zone de l'interface utilisateur à laquelle la force est appliquée correspond à la seconde zone, les un ou plusieurs processeurs sont configurés pour déterminer l'amplitude de chacune des une ou plusieurs lectures optiques sur la base d'un second modèle ayant un second paramètre d'échelle (k2) qui reflète une seconde variance de la force appliquée à l'interface utilisateur, la seconde variance étant différente de la première variance.

6. Dispositif informatique portable selon la revendication 5, dans lequel
la détermination de la localisation où la force est appliquée à l'interface utilisateur comprend l'utilisation d'une valeur de chacune des une ou plusieurs lectures optiques produites par les capteurs et des positions connues des capteurs pour estimer la localisation où la force est appliquée en appliquant une fonction de perte pour minimiser une différence entre une localisation réelle où la force est appliquée et une localisation estimée où la force est appliquée, dans lequel la fonction de perte comporte une fonction de densité de probabilité déterminant une probabilité qu'une lecture optique produite par un capteur optique soit corrompue par des bruits de mesure.

7. Dispositif informatique portable selon la revendication 6, dans lequel
les un ou plusieurs processeurs sont configurés pour appliquer la fonction de perte en réalisant une méthode de recherche sur grille bidimensionnelle ou une méthode de recherche par descente de gradient.

8. Dispositif informatique portable selon la revendication 1, dans lequel
a)
l'interface utilisateur comporte un écran non tactile (182) configuré pour afficher une pluralité d'éléments correspondant à une pluralité de fonctions sélectionnables (F3, F4) du dispositif informatique portable,
en réponse à un niveau de bruit des capteurs augmentant au-delà d'un seuil, les un ou plusieurs processeurs sont configurés pour commander l'écran non tactile afin d'arrêter l'affichage d'un ou de plusieurs éléments de la pluralité d'éléments, et
en réponse à la détermination que la localisation où la force est appliquée sur l'écran non tactile correspond à un premier élément restant de la pluralité d'éléments affichés sur l'écran non tactile, les un ou plusieurs processeurs sont en outre configurés pour exécuter une première fonction sélectionnable de la pluralité de fonctions sélectionnables qui correspond au premier élément restant, sur la base de la localisation déterminée ; et/ou
b)
l'interface utilisateur comporte une pluralité de faux boutons (184a, 184b) qui correspondent respectivement à une pluralité de fonctions sélectionnables (F3, F4) du dispositif informatique portable, et
en réponse à la détermination de la localisation où la force est appliquée à un premier faux bouton de la pluralité de faux boutons de l'interface utilisateur, les un ou plusieurs processeurs sont configurés pour exécuter une première fonction sélectionnable de la pluralité de fonctions sélectionnables qui correspond au premier faux bouton, sur la base de la localisation déterminée.

9. Dispositif informatique portable selon la revendication 1, dans lequel
les un ou plusieurs processeurs sont configurés pour identifier un geste de l'utilisateur sur la base des une ou plusieurs lectures optiques produites par les capteurs PPG, et
les un ou plusieurs processeurs sont configurés pour exécuter une fonction du dispositif informatique portable sur la base du geste de l'utilisateur identifié par les un ou plusieurs processeurs.

10. Dispositif informatique portable selon la revendication 9, comprenant en outre :
au moins l'un d'un accéléromètre (174) ou d'un gyroscope (176),
dans lequel
les un ou plusieurs processeurs sont configurés pour identifier le geste de l'utilisateur sur la base des une ou plusieurs lectures optiques produites par les capteurs PPG et des une ou plusieurs sorties de l'au moins un de l'accéléromètre ou du gyroscope.

11. Procédé mis en œuvre par ordinateur, comprenant :
la réception d'une ou de plusieurs lectures optiques (S1, S2, S3) produites par des capteurs (170) disposés sur un côté inférieur (110b) d'un boîtier (110) d'un dispositif informatique portable (100), lorsqu'une force est appliquée à une interface utilisateur (180) disposée sur un côté supérieur (110a) du boîtier, dans lequel le côté inférieur du boîtier est opposé au côté supérieur du boîtier et est configuré pour être en contact avec une partie de corps (102) d'un utilisateur lorsque le dispositif informatique portable est porté par l'utilisateur, dans lequel les capteurs comportent des capteurs de photopléthysmographie, PPG, (172) configurés pour surveiller une fréquence cardiaque de l'utilisateur lorsque le dispositif informatique portable est porté par l'utilisateur, et les capteurs PPG comportent une ou plusieurs diodes électroluminescentes et une pluralité de détecteurs (172a, 172b, 172c) ; et
la détermination, par un ou plusieurs processeurs (150) du dispositif informatique portable, d'une localisation où la force est appliquée à l'interface utilisateur sur la base des une ou plusieurs lectures optiques reçues produites par les capteurs PPG.

12. Procédé mis en œuvre par ordinateur selon la revendication 11, comprenant en outre :
l'exécution d'une ou de plusieurs fonctions (F3, F4) du dispositif informatique portable sur la base de la localisation où la force est appliquée à l'interface utilisateur tel que déterminé par les un ou plusieurs processeurs.

13. Procédé mis en œuvre par ordinateur selon la revendication 11, comprenant en outre :
l'affichage sur un écran (182) des instructions d'interface utilisateur indiquant à un utilisateur de calibrer le dispositif informatique portable en indiquant une zone de l'écran à laquelle la force doit être appliquée à l'écran.

14. Procédé mis en œuvre par ordinateur selon la revendication 13, comprenant en outre :
lorsque la zone de la force indiquée par l'utilisateur correspond à une première zone, la détermination d'une amplitude de chacune des une ou plusieurs lectures optiques sur la base d'un premier modèle ayant un premier paramètre d'échelle (k1) qui reflète une première variance de la force à appliquer à l'écran, et
lorsque la zone de la force indiquée par l'utilisateur correspond à une seconde zone, la détermination de l'amplitude de chacune des une ou plusieurs lectures optiques sur la base d'un second modèle ayant un second paramètre d'échelle (k2) qui reflète une seconde variance de la force à appliquer à l'écran, la première zone étant supérieure à la seconde zone et la première variance étant supérieure à la seconde variance.

15. Procédé mis en œuvre par ordinateur selon la revendication 11, la détermination de la localisation où la force est appliquée à l'interface utilisateur comprend l'utilisation d'une valeur de chacune des une ou plusieurs lectures optiques produites par les capteurs et des positions connues des capteurs pour estimer la localisation où la force est appliquée en appliquant une fonction de perte pour minimiser une différence entre une localisation réelle où la force est appliquée et une localisation estimée où la force est appliquée, dans lequel la fonction de perte comporte une fonction de densité de probabilité déterminant une probabilité qu'une lecture optique produite par un capteur optique soit corrompue par des bruits de mesure.
